# EUROPEAN PATENT SPECIFICATION

(11) **EP 4 301 279 B1**
(45) Date of publication and mention of the grant of the patent: **28.10.2026**
(21) Application number: 22710875.0
(22) Date of filing: 28.02.2022
(51) Int. Cl.: A61F 2/24, A61F 2/95, A61F 2/966, A61F 2/962

(54) **DELIVERY APPARATUS FOR IMPLANTING PROSTHETIC DEVICES**
FREISETZUNGSVORRICHTUNG ZUR IMPLANTATION VON PROTHESEN
APPAREIL DE DISTRIBUTION D'IMPLANTATION D'APPAREILLAGES PROTHÉTIQUES

(30) Priority: 01.03.2021 US 202163154956 P; 01.03.2021 US 202163154966 P
(43) Date of publication of application: 10.01.2024
(73) Proprietor: Edwards Lifesciences Corporation, Irvine, CA 92614 (US)
(72) Inventor: DESROSIERS, John, J., Irvine, CA 92614 (US)
(74) Representative: Eisenführ Speiser
(86) International application number: PCT/US2022/018093
(87) International publication number: WO 2022/187121

(56) References cited:
- US-A1- 2010 049 313
- US-A1- 2012 226 341
- US-A1- 2014 031 922

## Description

### CROSS REFERENCE TO RELATED APPLICATIONS

This application claims the benefit of U.S. Provisional Application No. 63/154,956, filed March 1, 2021, and U.S. Provisional Application No. 63/154,966, filed March 1, 2021.

### FIELD

The present disclosure relates generally to delivery apparatus and methods for implanting prosthetic devices and more particularly to delivery apparatus and method for implanting support structures and/or prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally invasive surgical approaches are used in various procedures to deliver prosthetic medical devices to locations inside the body that are not readily accessible by surgery or where access without surgery is desirable.

In one specific example, a prosthetic valve can be mounted in a crimped state on the distal end of a delivery apparatus and advanced through the patient's vasculature (e.g., through a femoral artery and the aorta) until the prosthetic valve reaches the implantation location in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery apparatus so that the prosthetic valve can self-expand to its functional size.

In some cases, it may not be possible to secure the prosthetic valve to the native valve annulus, for example, if the native valve annulus is too large or if the geometry of the native valve is too complex to allow secure implantation of the valve. One approach in these cases is to first deploy a docking station at the implantation location and then install the prosthetic valve in the docking station. The docking station can be selected to provide the necessary interface to anchor the prosthetic valve within the native valve annulus. Desirably, the docking station can be delivered to the implantation location with a minimally invasive procedure, which would allow the docking station to be deployed within the same procedure used to deliver the prosthetic valve.

US 2010/0049313 A1 discloses a heart-valve delivery apparatus for delivering a prosthetic heart valve via a patient's vasculature. The heart-valve delivery apparatus, comprises a catheter comprising a flexible torque shaft adapted to extend through the patient's vasculature, the torque shaft having a distal end portion coupled to the prosthetic valve. Moreover, the heart-valve delivery apparatus comprises a valve sheath configured to receive the valve in a radially compressed state when coupled to the distal end portion of the torque shaft for delivery to the heart through the patient's vasculature. Rotation of the torque shaft is effective to cause relative longitudinal movement between the sheath and the valve to advance the valve from the sheath for deployment in the heart.
Further, US 2012/226341 A1 relates to a modular catheter system including a sheath projecting distally from a delivery catheter having a main body module, an inner core module carrying a stent thereon, the inner core being axially movable through the main body of the delivery catheter and the delivery catheter sheath, a handle member supported by the main body of the delivery catheter, the handle member being selectively axially engageable with the inner core such that the handle member and the inner core move together in an axial direction when the handle member is engaged with the inner core; and an adjustment member supported by the main body, the adjustment member being configured such that rotation of the adjustment member causes the adjustment member to move axially along the main body by either axially sliding the handle member relative to the main body or by rotating the adjustment member..

### SUMMARY

Disclosed herein are examples of a delivery apparatus that can be used to deliver a prosthetic implant, such as a docking station, to an implantation location within a patient's body. The delivery apparatus includes a handle and a shaft assembly coupled to the handle. The shaft assembly includes an outer shaft and an inner shaft extending through a lumen of the outer shaft. A carriage within the handle is coupled to the outer shaft and movable relative to the handle to displace the outer shaft axially and relative to the handle. Movement of the carriage can displace the outer shaft between an extended position to capture the prosthetic implant and a retracted position to expose the prosthetic implant. **In** some examples, the carriage includes a stepdown shoulder that together with a proximal end of the outer shaft form a gland. A seal member may be disposed within the gland to seal between the carriage and the shaft assembly. The stepdown shoulder in the carriage allows the carriage to be molded as a single piece, simplifying both manufacture and assembly of the delivery apparatus. **In** some examples, the inner shaft includes one or more fluid ports that fluidly connect a lumen of the inner shaft to the lumen of the outer shaft, allowing both lumens to be flushed from a single injection port. **In** some examples, a frame connector is provided that couples the implant device to the inner shaft. The frame connector includes a recess that is configured to receive a connector tab on the implant device. The recess has at least one undercut wall that translates tensile force applied to the connector tab to radial force acting on the connector tab, which can improve retention characteristics of the prosthetic implant prior to deploying the prosthetic implant at the implantation location and particularly during recapture of the prosthetic implant.

**In** one representative example, a delivery apparatus comprises a handle body, a carriage member, an outer shaft, an inner shaft, and a seal member. The handle body includes a proximal end, a distal end, a longitudinal axis extending between the proximal end and the distal end, and a cavity disposed between the proximal end and the distal end. The carriage member is disposed within the cavity and axially movable relative to the handle body in a direction parallel to the longitudinal axis of the handle body. The carriage member has an internal surface defining an internal bore and a gland shoulder integrally formed with the internal surface and defining a stepdown transition in the internal bore. The outer shaft includes a proximal end positioned within the internal bore of the carriage member and in opposing relation to the gland shoulder. The proximal end of the outer shaft, the gland shoulder of the carriage member, and a portion of the internal surface of the carriage member adjacent to the stepdown transition define an annular groove. The inner shaft extends through a lumen of the outer shaft and is fixed relative to the handle body. The seal member is disposed around the inner shaft and within the annular groove and is positioned to form a seal between the carriage member and the inner shaft and at the proximal end of the outer shaft.

**In** another representative example, a delivery assembly comprises the preceding delivery apparatus and an expandable docking station releasably coupled to the delivery apparatus, wherein the expandable docking station is configured to receive a prosthetic heart valve.

**In** another representative example, a method comprises inserting a distal end of the preceding delivery assembly into a vasculature of a patient, advancing the delivery assembly through the vasculature of the patient to position the expandable docking station at a select implantation location, and moving the carriage member relative to the handle to release the expandable docking station from the delivery apparatus.

**In** another representative example, a handle for a prosthetic implant delivery apparatus comprises a handle body and a carriage member. The handle body includes a longitudinal axis and a cavity extending along the longitudinal axis. The carriage member is disposed within the cavity and axially movable relative to the longitudinal axis of the handle body. The carriage member comprises a carriage body having an internal surface defining an internal bore and a gland shoulder integrally formed with the internal surface and defining a stepdown transition in the internal bore. The gland shoulder and a portion of the internal surface adjacent to the stepdown transition form a portion of an annular groove configured to receive a seal member.

**In** another representative example, a carriage for a prosthetic implant delivery apparatus comprises a one-piece molded body having an internal surface defining an internal bore and a gland shoulder integrally formed with the internal surface and defining a stepdown transition in the internal bore. The gland shoulder and a portion of the internal surface adjacent to the stepdown transition form a portion of an annular groove configured for receiving a seal member.

In another representative example, a method of forming a component of a prosthetic implant delivery apparatus comprises securing a core pin within a mold cavity and injecting a thermoplastic material into the mold cavity to form a molded body having an internal surface defining an internal bore and a gland shoulder integrally formed with the internal surface and defining a first stepdown transition in the internal bore.

In another representative example, a method of forming a component of a prosthetic implant delivery apparatus comprises securing a core pin within a mold cavity and injecting a thermoplastic material into the mold cavity to form a mold body having an internal surface defining an internal bore, a gland shoulder integrally formed with the internal surface and defining a stepdown transition in the internal bore, and a locating shoulder integrally formed with the internal surface and axially displaced from the gland shoulder.

In another representative example, a delivery apparatus comprises a handle body, an outer shaft, an inner shaft, and an injection port. The handle body includes a longitudinal axis and a cavity extending along the longitudinal axis. The outer shaft includes a proximal end positioned within the cavity. The outer shaft has a first lumen. The inner shaft extends through the first lumen of the outer shaft. The inner shaft has a second lumen and one or more fluid ports that fluidly connect the second lumen to the first lumen. The injection port is fluidly connected to the second lumen of the inner shaft, and both the first and second lumens are flushable with a fluid through the injection port.

In another representative example, a delivery assembly comprises the preceding delivery apparatus and an expandable docking station for an expandable valve releasably coupled to the delivery apparatus.

In another representative example, a method comprises inserting a distal end of the preceding delivery assembly into a vasculature of a patient, advancing the distal end of the delivery assembly through the vasculature of the patient to position the expandable docking station at a select implantation location, and moving the carriage member relative to the handle to release the expandable docking station from the delivery apparatus.

In another representative example, a method comprises providing a reinforced tube comprising an inner layer, a reinforcement layer disposed over the inner layer, and an outer layer disposed over the reinforcement layer. The method includes ablating the reinforced tube at one or more locations to form one or more fluid ports in the reinforced tube.

**In** another representative example, a method comprises disposing a cover tube having one or more windows over a reinforced tube and ablating the reinforced tube at one or more locations exposed through the one or more windows to form one or more fluid ports in the reinforced tube.

**In** another representative example, a shaft assembly for a prosthetic implant delivery apparatus comprises an outer shaft having a first lumen and an inner shaft extending through the first lumen. The inner shaft comprises a reinforced tube having a second lumen and one or more fluid ports that fluidly connect the second lumen to the first lumen. The inner shaft further includes a cover tube disposed over the reinforced tube. The cover tube has one or more windows positioned to expose the one or more fluid ports to the first lumen.

**In** another representative example, a delivery apparatus comprises an elongate shaft and a frame connector. The elongate shaft has a proximal end portion and a distal end portion. The proximal end portion is configured to be disposed outside of a patient's body during a delivery procedure, and the distal end portion is configured to be disposed inside a patient's body during the delivery procedure. The frame connector is coupled to the distal end portion of the elongate shaft and configured for releasably coupling a prosthetic implant to the delivery apparatus. The frame connector comprises a connector body having an exterior with an exterior surface and a recess. The recess comprises a first slot portion having a first width, a second slot portion having a second width that is larger than the first width, a recess floor, and opposed first and second side walls extending from the recess floor to the exterior surface and connected to the first slot portion and the second slot portion. At least a first portion of each of the first and second side walls connected to the second slot portion includes an undercut from the exterior surface to the recess floor.

**In** another representative example, a frame connector for a prosthetic implant delivery apparatus comprises a connector body having an exterior with an exterior surface, a recessed surface, and opposed first and second side walls. The recessed surface is spaced radially inwardly relative to the exterior surface and comprises a first slot portion having a first width and a second slot portion having a second width that is larger than the first width. The opposed first and second side walls extend radially from the recessed surface to the exterior surface and are connected to the first slot portion and the second slot portion. At least a first portion of each of the first and second side walls connected to the second slot portion forms an angle relative to the recessed surface within a range of 75-89.9 degrees.

In another representative example, a delivery assembly comprises a self-expandable docking station and a frame connector. The self-expandable docking station comprises at least one connector tab having a flared portion. The frame connector comprises a connector body having at least one recess to receive and retain the at least one connector tab. The at least one recess comprises a slot portion to receive the flared portion, a recess floor, and opposed first and second side walls connected to the slot portion and the recess floor. At least a portion of each of the first and second side walls forms an angle relative to the recess floor within a range of 75-89.9 degrees.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 is an elevation view of a portion of a frame of a docking station in a radially-expanded state.
FIG. 2 is a perspective view of the frame of FIG. 1 in a radially-compressed state.
FIG. 3 is a perspective view of a docking station including the frame of FIG. 1.
FIG. 4 is a cut-away view of the docking station of FIG. 3 deployed at an implantation location within a patient's anatomy, which is depicted schematically in cross-section, and with a prosthetic heart valve deployed therein.
FIG. 5A is a perspective view of a delivery apparatus for deploying a docking station.
FIG. 5B illustrates the docking station of FIG. 3 disposed around a distal portion of the delivery apparatus of FIG. 5A.
FIG. 6A is an elevation view of a distal portion of the delivery apparatus of FIG. 5A with an outer shaft of the delivery apparatus in a retracted position.
FIG. 6B is an elevation view of a distal portion of the delivery apparatus of FIG. 5A with an outer shaft of the delivery apparatus in an extended position and cut away to show an encapsulated docking station.
FIGS. 6C-6F illustrate stages in deployment of the docking station of FIG. 3 from the delivery apparatus of FIG. 5A.
FIG. 7A is a perspective view of a handle portion of the delivery apparatus illustrated in FIG. 5A.
FIGS. 7B and 7C are perspective views of the handle portion of FIG. 7A with a portion of the handle cut away to show various internal components.
FIGS. 8A and 8B are perspective views of a carriage member of the handle portion of FIG. 7A.
FIG. 8C is a cross-sectional view of the carriage member of FIGS. 8A and 8B.
FIG. 9 is a cross-sectional view of a head portion of the carriage member of FIGS. 8A and 8B.
FIG. 10 is a cross-sectional view of the carriage member of FIGS. 8A and 8B with a proximal portion of a shaft assembly extending through the carriage member.
FIG. 11A is a cross-sectional view of the handle portion of FIG. 7A, taken along a plane intersecting line 1 1A-11A as depicted in FIG. 7A.
FIG. 11B is a cross-sectional view of the handle portion of FIG. 7A, taken along line 11B-11B as depicted in FIG. 11A.
FIG. 12A is a cross-sectional view of a proximal portion of the shaft assembly coupled to the handle portion of FIG. 7A with a portion of the shaft assembly cut away to show fluid ports in an inner shaft of the shaft assembly.
FIG. 12B is a cross-sectional view of a portion of the inner shaft of the shaft assembly illustrated in FIG. 12A.
FIG. 12C is an enlarged view of the region 12C as depicted in FIG. 12A.
FIGS. 13A and 13B are elevation views of a frame connector.
FIGS. 14 is a perspective view of the frame connector of FIGS. 13A and 13B with a cut-away plane taken along line 14-14 as depicted in FIG. 13A.
FIG. 15 illustrates the frame connector of FIGS. 13A and 13B with a connector tab of a docking station retained in a recess of the frame connector.
FIG. 16A is a perspective view of the frame connector of FIGS. 13A and 13B with a cut-away plane taken along line 16A-16A as depicted in FIG. 13A.
FIG. 16B is a cross-sectional view of the frame connector of FIGS. 13A and 13B at the cut-away plane shown in FIG. 16A.
FIG. 17A is a perspective view of the frame connector of FIGS. 13A and 13B with a cut-away plane taken along line 17A-17A as depicted in FIG. 13A.
FIG. 17B is a cross-sectional view of the frame connector of FIGS. 13A and 13B at the cut-away plane shown in FIG. 17A.
FIG. 18 is a cross-sectional view of a distal portion of a delivery apparatus illustrating the frame connector of FIGS. 13A and 13B connected to an inner shaft of the shaft assembly of FIGS. 5A and 5B.
FIG. 19 is an elevation view of a distal portion of the delivery apparatus of FIG. 5A with an outer shaft of the delivery apparatus in an extended position and cut away to show a docking station restrained by the outer shaft and the frame connector of FIGS. 13A and 13B.
FIG. 20 is a rotated view of the distal portion of the delivery apparatus depicted in FIG. 19 with the frame connector cut away to show engagement with connector tabs of a docking station.
FIG. 21 illustrates radial deflection of the connector tabs of the docking station of FIGS. 19 and 20 in response to axial tension applied to the connector tabs.

### DETAILED DESCRIPTION

### General Considerations

For the purposes of this description, certain specific details are set forth herein in order to provide a thorough understanding of disclosed examples. In some cases, as will be recognized by one skilled in the art, the disclosed examples may be practiced without one or more of these specific details, or may be practiced with other methods, structures, and materials not specifically disclosed herein. In some instances, well-known structures and/or processes associated with prosthetic valves and delivery apparatuses have been omitted to avoid obscuring novel and non-obvious aspects of the disclosed examples.

The disclosed technology is described via examples and implementations. The sequential order of the acts in any process described herein may be rearranged, unless the context clearly dictates otherwise, such as if one act requires the result of another act as input.

In the interest of conciseness, and for the sake of continuity in the description, same or similar reference characters may be used for same or similar elements in different figures, and description of an element in one figure will be deemed to carry over when the element appears in other figures with the same or similar reference character. In some cases, the term "corresponding to" may be used to describe correspondence between elements of different figures. In an example usage, when an element in a first figure is described as corresponding to another element in a second figure, the element in the first figure is deemed to have the characteristics of the other element in the second figure, and vice versa, unless stated otherwise.

The word "comprise" and derivatives thereof, such as "comprises" and "comprising", are to be construed in an open, inclusive sense, that is, as "including, but not limited to". The singular forms "a", "an", "at least one", and "the" include plural referents, unless the context dictates otherwise. The term "and/or", when used between the last two elements of a list of elements, means any one or more of the listed elements. The term "or" is generally employed in its broadest sense, that is, as meaning "and/or", unless the context clearly dictates otherwise.

The term "coupled" without a qualifier generally means physically coupled or linked and does not exclude the presence of intermediate elements between the coupled elements absent specific contrary language. The term "plurality" or "plural" when used together with an element means two or more of the element. Directions and other relative references (e.g., inner and outer, upper and lower, above and below, left and right, and proximal and distal) may be used to facilitate discussion of the drawings and principles herein but are not intended to be limiting.

### Introduction to the Disclosed Technology

This disclosure describes a plurality of delivery apparatus that can be used to deliver prosthetic implants such as docking stations and/or prosthetic heart valves to an implantation location within a patient's anatomy. The delivery apparatus includes a shaft assembly coupled to a handle, which controls operations of the delivery apparatus. A prosthetic implant can be encapsulated within a distal end portion of one of the shafts of the shaft assembly for delivery to the implantation location.

The shaft assembly includes an outer shaft that is movable between an extended position to encapsulate a prosthetic implant loaded onto the delivery apparatus and a retracted position to expose the prosthetic implant for deployment at the implantation location. A carriage member is included in the handle to move the outer shaft between the retracted and extended positions. The shaft assembly includes an inner shaft that extends through the lumen of the outer shaft.

**In** certain examples, the carriage member and the outer shaft form a gland or annular groove to hold a seal member. **In** certain examples, the inner shaft includes one or more fluid ports that together with the seal member disposed within the carriage member allow the inner shaft and the outer shaft to be flushed with fluid from a single injection port.

**In** certain examples, the inner shaft can carry a frame connector having one or more recesses to receive one or more connector tabs of the prosthetic implant and thereby axially restrain the prosthetic implant. **In** certain examples, the recesses have undercut walls that translate tensile force applied to the connector tabs to radial force acting on the connector tabs, which can help maintain engagement of the connector tabs with the recesses during recompression and/or retrieval of the prosthetic implant.

### Examples of the Disclosed Technology

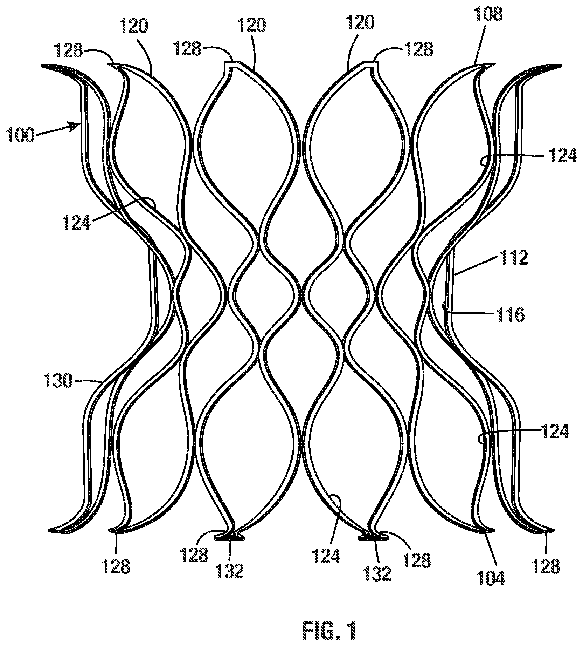

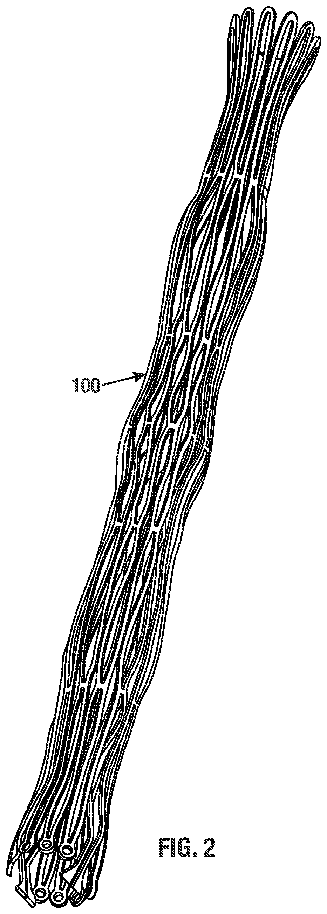

Turning now to the drawings, FIG. 1 illustrates an exemplary implementation of a frame 100 (or stent) that can form a body of a docking station. The frame 100 has a first end 104 and a second end 108. In some examples, the first end 104 can be an inflow end, and the second end 108 can be an outflow end. In other examples, the first end 104 can be an outflow end, and the second end 108 can be an inflow end. The terms "inflow" and "outflow" are related to the normal direction of blood flow (e.g., antegrade blood flow) through the frame. In the unconstrained, expanded state of the frame 100 shown in FIG. 1, a relatively narrower portion (or waist) 112 of the frame 100 between the first end 104 and the second end 108 forms a valve seat 116. The frame 100 can be compressed (as illustrated in FIG. 2) for delivery to an implantation location by a delivery apparatus.

Although the docking stations, delivery apparatus, prosthetic heart valves, and/or methods are described herein with respect to a particular implantation location (e.g., a pulmonary valve) and/or a particular delivery approach (e.g., transfemoral), the device and methods disclosed herein can be adapted to various other implantation locations (e.g., an aortic valve, a mitral valve, and a tricuspid valve) and/or delivery approaches (e.g., transapical, transseptal, etc.).

In the example illustrated by FIG. 1, the frame 100 includes a plurality of struts 120 arranged to form cells 124. The ends of the struts 120 form apices 128 at the ends of the frame 100. One or more of the apices 128 can include a connector tab 132. The portions of the struts 120 between the apices 128 and the valve seat 116 (or the waist 112) form a sealing portion 130 of the frame 100. In the unconstrained, expanded state of the frame 100 illustrated in FIG. 1, the apices 128 extend generally radially outward and are radially outward of the valve seat 116.

The frame 100 can be made of a highly resilient or compliant material to accommodate large variations in the anatomy. For example, the frame 100 can be made of a flexible metal, metal alloy, polymer, or an open cell foam. An example of a highly resilient metal is Nitinol, which is a metal alloy of nickel and titanium, but other metals and high resilient or compliant non-metal materials can be used. The frame 100 can be self-expanding, manually expandable (e.g., expandable via a balloon), or mechanically expandable. A self-expanding frame can be made of a shape memory material, such as, for example, Nitinol. In this manner, the frame can be radially compressed as depicted in FIG. 2 (e.g., via a crimping device) and can radially expand to the configuration depicted in FIG. 1.

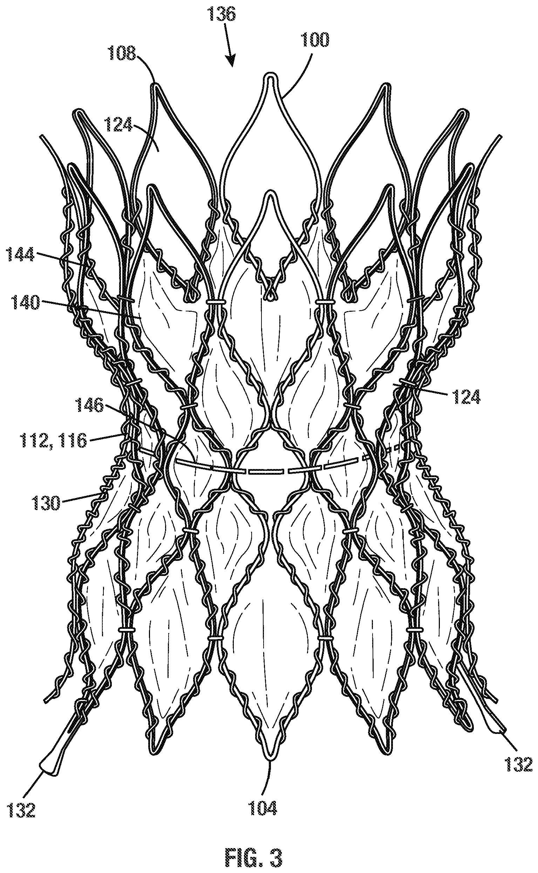

FIG. 3 illustrates an exemplary docking station 136 including the frame 100 and an impermeable material 140 disposed within the frame. The impermeable material 140 is attached to the frame 100 (e.g., by sutures 144). In the example illustrated by FIG. 3, the impermeable material 140 covers at least the cells 124 in the sealing portion 130 of the frame 100. The seal formed by the impermeable material 140 at the sealing portion 130 can help funnel blood flowing into the docking station 136 from the proximal inflow end 104 to the valve seat 116 (and the valve once installed in the valve seat). One or more rows of cells 124 proximate to the distal outflow end 108 can be open.

The impermeable material 140 can be a fabric that is impermeable to blood. A variety of biocompatible materials can be used as the impermeable material 140, such as, for example, foam or a fabric that is treated with a coating that is impermeable to blood, a polyester material, or a processed biological material, such as pericardium. In one particular example, the impermeable material 140 can be polyethylene terephthalate (PET).

The docking station 136 may include a band 146 that extends around the waist 112 (or that is integral to the waist) of the frame 100. The band 146 can constrain expansion of the valve seat 116 to a specific diameter in the deployed state to enable the valve seat 116 to support a specific valve size. The band 146 can take on a wide variety of different forms and can be made of a wide variety of different materials. For example, the band 146 can be made of PET, one or more sutures, fabric, metal, polymer, a biocompatible tape, or other relatively nonexpanding materials known in the art and that can maintain the shape of the valve seat 116.

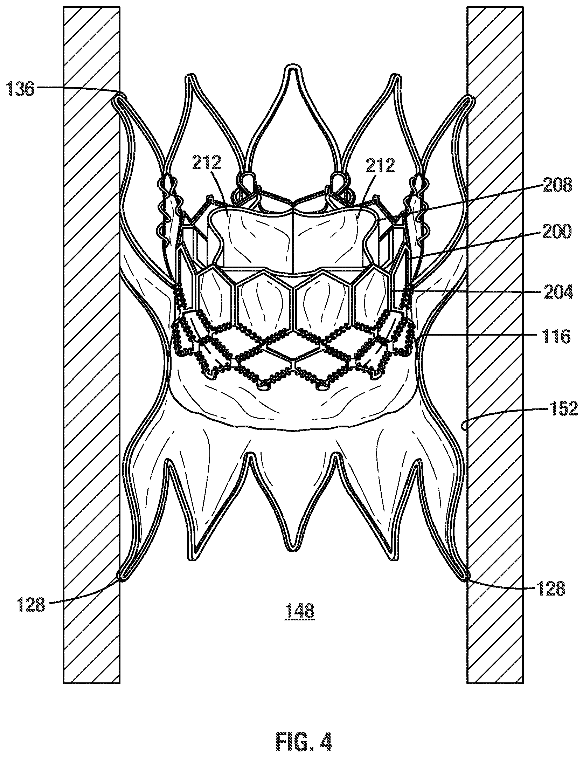

FIG. 4 illustrates the docking station 136 in a deployed state within a native valve annulus 148. As can be seen, the frame 100 of the docking station 136 is in an expanded condition, with the end portions of the frame pressed against the inner surface 152 of the native valve annulus. The band 146 (shown in FIG. 3) can maintain the valve seat 116 at a constant or substantially constant diameter in the expanded condition of the frame 100. FIG. 4 also shows a prosthetic valve 200 deployed within the docking station 136 and engaged with the valve seat 116 of the docking station 136. The prosthetic valve 200 can be implanted by first deploying the docking station 136 at the implantation location and then installing the prosthetic valve within the docking station.

The prosthetic valve 200 can be configured to replace a native heart valve (e.g., aortic, mitral, pulmonary, and/or tricuspid valves). In one example, the prosthetic valve 200 can include a frame 204 and a valvular structure 208 disposed within and attached to the frame 204. The valvular structure 208 can include one or more leaflets 212 that cycle between open and closed states during the diastolic and systolic phases of the heart. The frame 204 can be made of the frame materials described for the frame 100 of the docking station 136. The leaflets 212 can be made in whole or in part from pericardial tissue (e.g., bovine pericardial tissue), biocompatible synthetic materials, or various other suitable natural or synthetic materials known in the art.

The docking station 136 is not limited to use with the particular example of the prosthetic valve 200 illustrated in FIG. 4. For example, mechanically expandable prosthetic valves such as described in U.S. Patent Publication Nos. 2018/0153689 and 2019/0060057; U.S. Patent Application No. 62/869,948; and International Application No. PCT/US2019/056865 may be installed in the docking station 136.

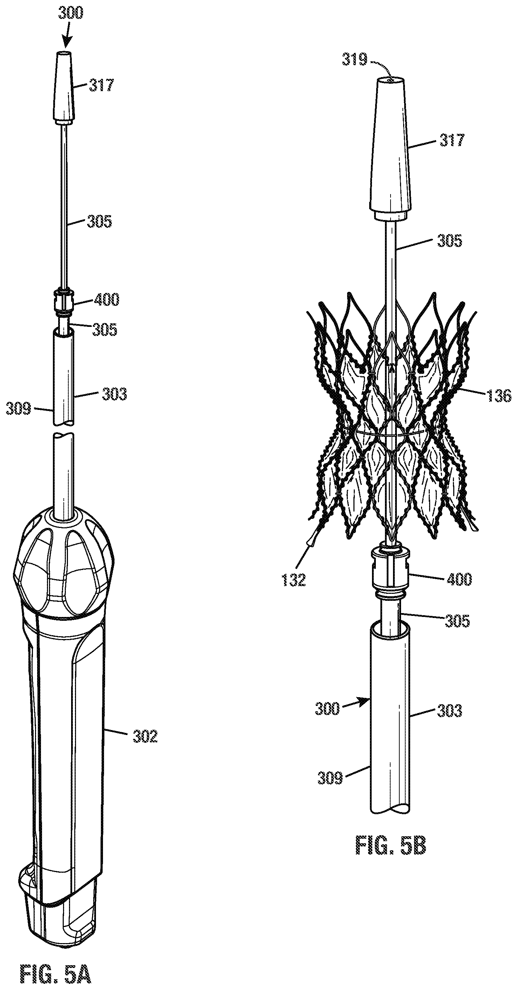

FIG. 5A illustrates an exemplary delivery apparatus 300 that can be used to deliver the docking station to an implantation location. The delivery apparatus 300 generally includes a handle 302 and a shaft assembly 303 coupled to the handle 302 and extending distally from the handle 302. The shaft assembly 303 includes an inner shaft 305 and an outer shaft 309. The inner shaft 305 extends through a lumen of the outer shaft 309.

In the example illustrated by FIG. 5A, a frame connector 400 is coupled to the inner shaft 305. The docking station 136 can be disposed around a portion of the inner shaft 305 extending distally from the frame connector 400, as shown in FIG. 5B. In one example, the frame connector 400 includes one or more recesses that can receive one or more connector tabs 132 at the proximal end of the docking station 136 and thereby axially restrain the docking station 136.

A nosecone 317 can be attached to a distal end of the inner shaft 305. The nosecone 317 includes a central opening 319 for receiving a guidewire. As such, a proximal end of the guidewire can be inserted into the central opening 319 and through the inner shaft 305, and a distal end portion of the delivery apparatus 300 can be advanced over the guidewire through a patient's vasculature and to an implantation location. The guidewire can pass through the nosecone 317 into the inner shaft 305 during advancing of the delivery apparatus through a patient's vasculature.

The handle 302 can be operated to move the outer shaft 309 relative to the inner shaft 305, generally between an extended position and a retracted position. The handle 302 can be extended to slide the outer shaft 309 over the frame connector 400 and over any docking station coupled to the frame connector 400 to encapsulate the docking station within the outer shaft 309. As the outer shaft 309 slides over the docking station 136, the outer shaft 309 can compress the docking station 136 such that the docking station is encapsulated within the outer shaft 309 in the compressed state. In the fully extended position, a distal end of the outer shaft 309 can abut a proximal end of the nosecone 317 such that there are no gaps in the delivery assembly. Additionally (or alternatively), a crimping device can be used to radially compress the docking station such that it can be inserted into the outer shaft of the delivery apparatus.

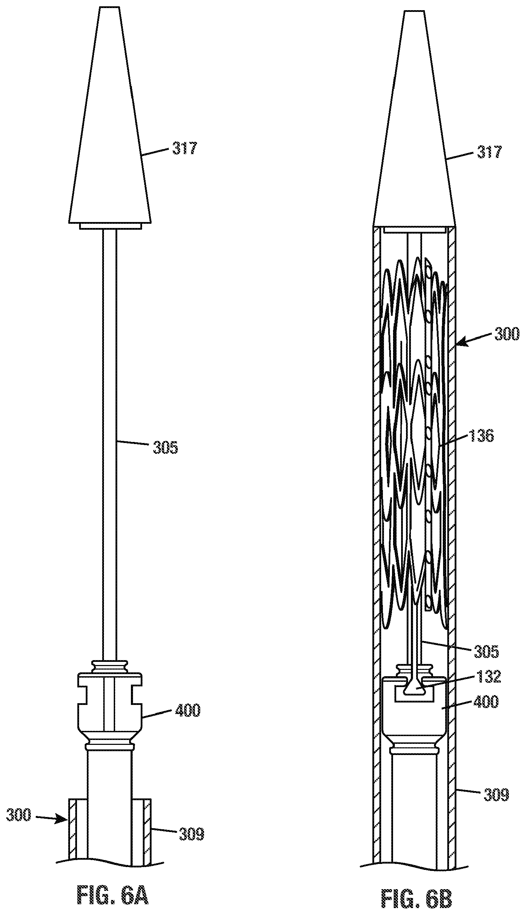

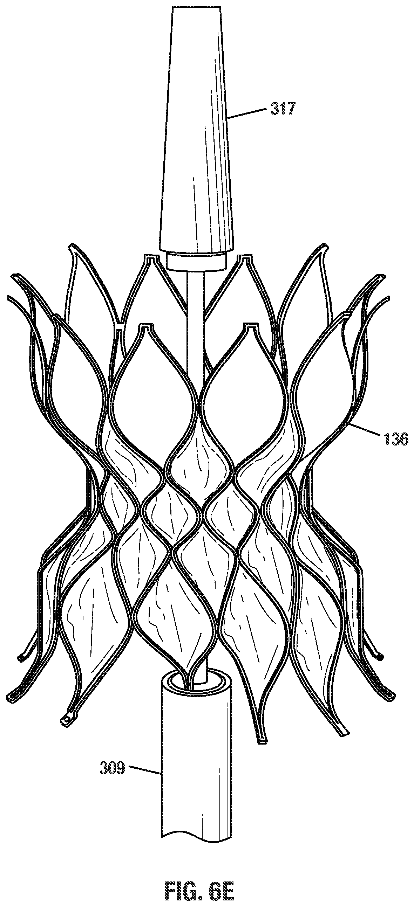

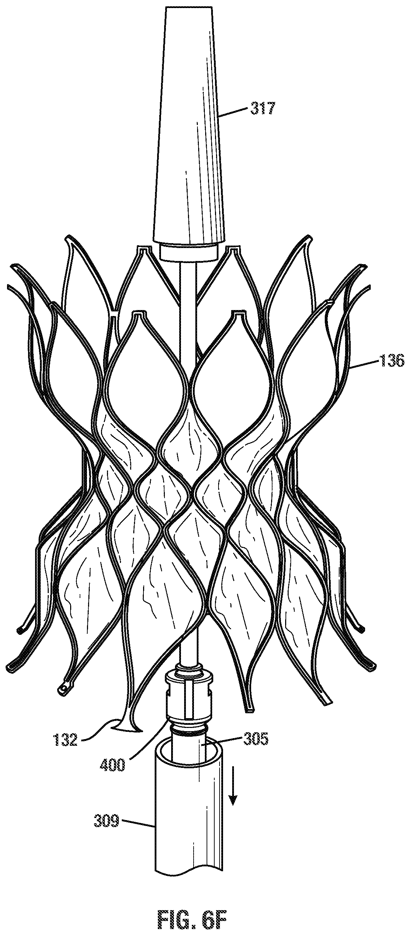

FIGS. 6A-7D illustrate a method of deploying a docking station at an implantation location within an anatomy. For purposes of illustration, the patient's anatomy is omitted. In FIG. 6A, the method includes retracting the outer shaft 309 by the handle of the delivery apparatus to allow loading of the docking station 136 onto the inner shaft 305. In FIG. 6B, the method includes disposing the docking station 136 around the inner shaft 305 and engaging each of the connector tabs 132 of the docking station 136 with the frame connector 400. The method also includes positioning the outer shaft 309 over the docking station such that the docking station is encapsulated therein. This can be accomplished by manipulating the handle of the delivery apparatus. As shown in FIG. 6B, the distal end of the outer shaft 309 abuts the proximal end of the nosecone 317. The method includes inserting the delivery apparatus, from the nosecone 317 end, into a patient's vasculature and advancing the delivery apparatus through the patient's vasculature to the implantation location.

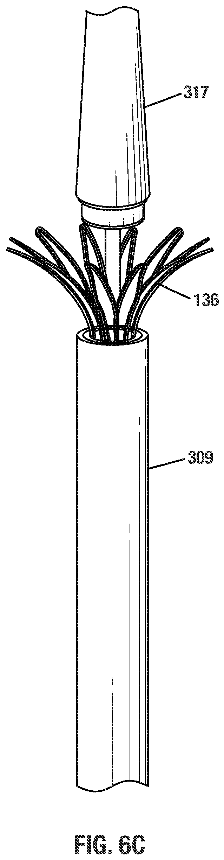

At the implantation location, the method includes retracting the outer shaft 309 by the handle of the delivery apparatus to expose the docking station 136. FIGS. 6C-6F show different stages of retracting the outer shaft 309. As can be seen, in cases where the docking station 136 is self-expanding, the docking station 136 gradually emerges from the outer shaft 309 and gradually expands from the compressed state as the outer shaft 309 is retracted. When the outer shaft 309 is sufficiently retracted, the connector tabs 132 disengage from the frame connector 400. Once the docking station 136 is disengaged from the frame connector 400, the docking station 136 can radially expand to engage the anatomy.

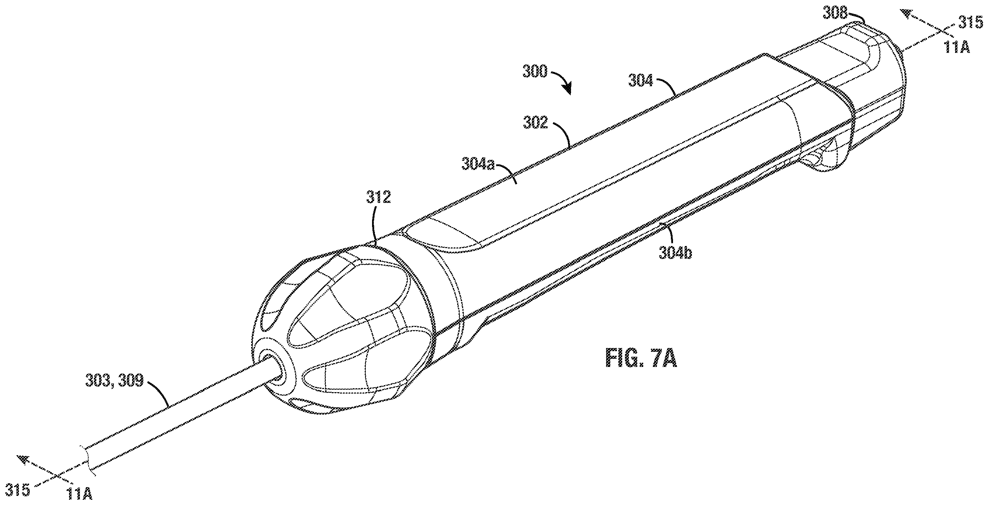

FIGS. 7A-7C illustrate an exemplary implementation of the handle 302 of the delivery apparatus. The handle 302 includes a handle body 304 and a deployment mechanism 306 coupled to and partially disposed within the handle body. The handle body 304 includes a proximal end 308, a distal end 312, and a cavity 316 extending from the proximal end 308 to the distal end 312. The handle 302 includes a longitudinal axis 315 extending from the proximal end 308 to the distal end 312. The longitudinal axis 315 defines the axial direction of the handle.

The handle body 304 can be a single piece body with the cavity 316. Alternatively, the handle body 304 can have two body pieces 304a, 304b that can be assembled together to form the cavity 316. For example, the first body piece 304b may have snap hooks 307 that snap into complementary recesses in the second body piece 304a.

The deployment mechanism 306 of the handle 302 includes a carriage member 500 and a drive member 320. The carriage member 500 is disposed within the cavity 316 and movable relative to the handle body 304 in the axial direction. The drive member 320 engages with the carriage member 500 and is movable (e.g., rotatable) relative to the handle body 304 to adjust the axial position of the carriage member 500 relative to the handle body 304.

Proximal portions of the shafts 305, 309 are inserted into the cavity of the handle body 304. A proximal end portion of the outer shaft 309 of the shaft assembly 303 can be coupled to the carriage member 500 (e.g., by fasteners, adhesive, and/or other means for coupling) such that movement of the carriage member 500 relative to the handle body 304 causes movement of the outer shaft 309 between the extended and retracted positions.

A proximal portion of the inner shaft 305 extends through a lumen 313 of the outer shaft 309 into a proximal portion of the cavity 316 and is coupled to the handle body 304. The inner shaft 305 can be fixed relative to the handle body 304 such that the inner shaft 305 is stationary while the outer shaft 309 moves relative to the handle body 304.

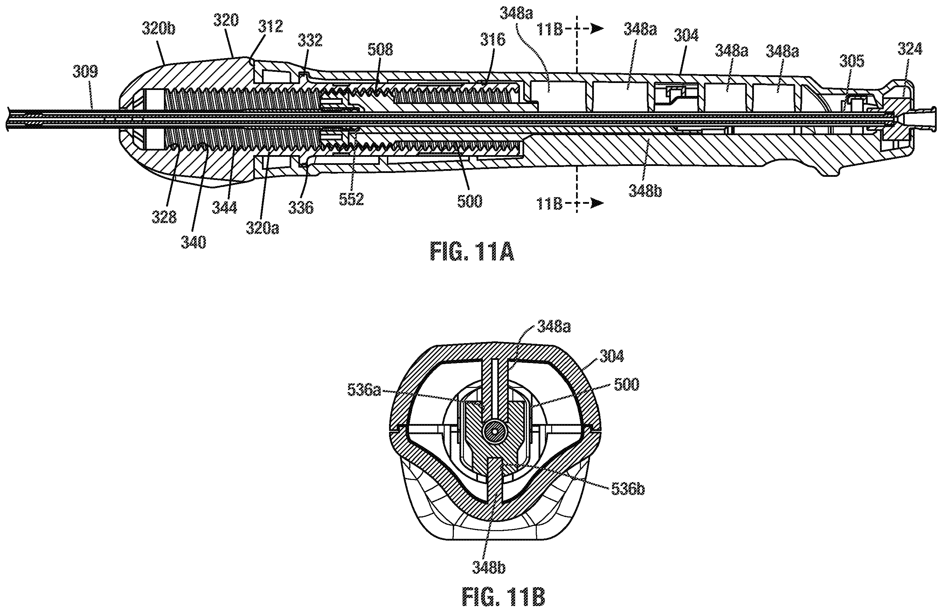

In the example illustrated by FIGS. 7A-7C, an injection port 324 is mounted at an opening at the proximal end 308 of the handle body 304. The injection port 324 can be, for example, a Luer fitting. A proximal end of the inner shaft 305 can be inserted into the injector port 324 (shown in FIG. 11A) and secured to the injection port 324 (e.g., by bonding). In some cases, the attachment of the inner shaft 305 to the injection port 324 can serve the purpose of fixing the inner shaft 305 relative to the handle body 304.

The injection port 324 can be used to inject flushing fluid, such as saline, into the lumen of the inner shaft 305. In some cases, the inner shaft 305 can include one or more fluid ports 311 through which the injected fluid exits the inner shaft 305 and enters the lumen 313 of the outer shaft 309, thereby allowing flushing of the lumens of the inner shaft 305 and outer shaft 309 from a single injection port.

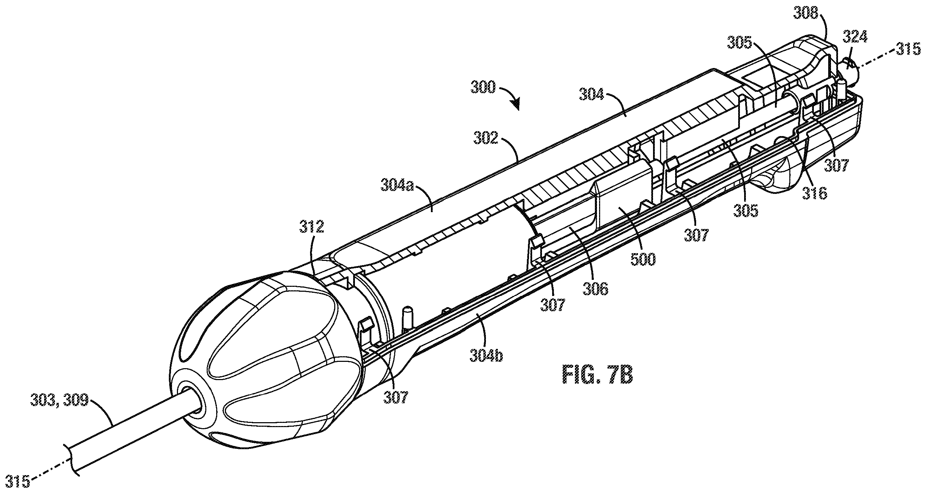

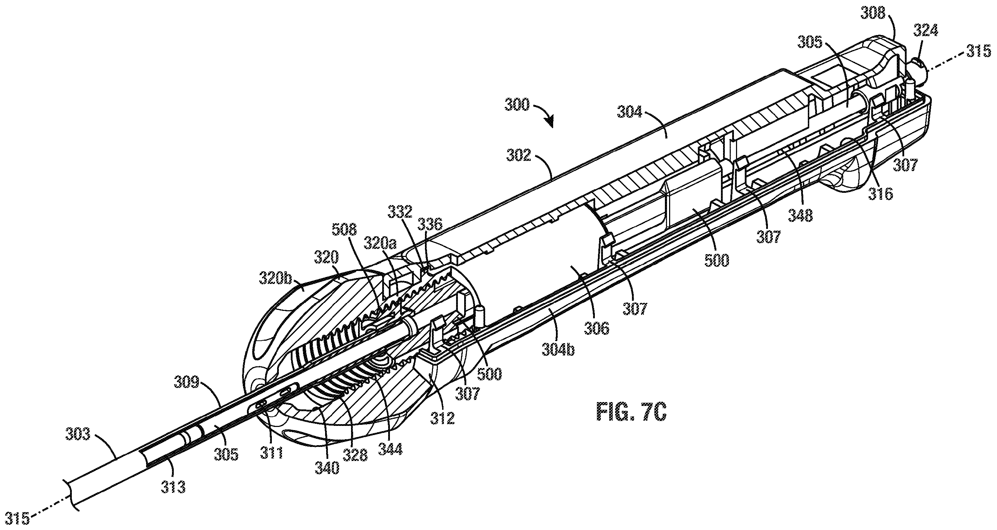

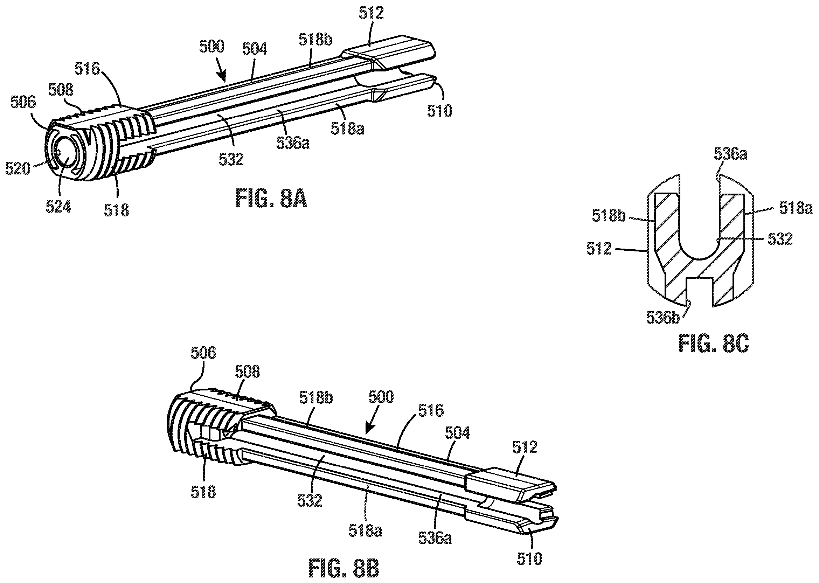

FIGS. 8A-8C illustrate an exemplary implementation of the carriage member 500. The carriage member 500 includes a carriage body 504 having a distal end 506 and a proximal end 510. The carriage body 504 has a head portion 508 and a stem portion 512 between the distal end 506 and the proximal end 510. The carriage body 504 can be formed (e.g., molded) as a single, unitary component. Preferably, the carriage body 504 has enough rigidity to support a portion of the shaft assembly received within the handle body 304 (shown in FIGS. 7B and 7C).

The head portion 508 of the carriage body 504 has an external surface 516. External threads 518 are formed on the portion of the external surface 516 at opposite sides of the head portion 508. The external threads 518 can engage a complementary internal thread in the drive member 320 (shown in FIGS. 7B and 7C) of the handle. The head portion 508 has an internal surface 520 that defines an internal bore 524 configured to receive a portion of the shaft assembly.

The stem portion 512 includes a central opening 532, which is longitudinally aligned with and connected to the internal bore 524 of the head portion 508, forming a passage extending along the entire length of the carriage body 504. Longitudinal slots 536a, 536b (or guide members) are formed on opposite sides of the stem portion 512. The longitudinal slot 536a may be connected to the central opening 532 (or to the passage formed by the bore 524 and central opening 532) as illustrated in FIG. 8C. The longitudinal slots 536a, 536b can receive complementary guide members 348a, 348b (shown in FIGS. 11A and 11B) within the elongated cavity of the handle body.

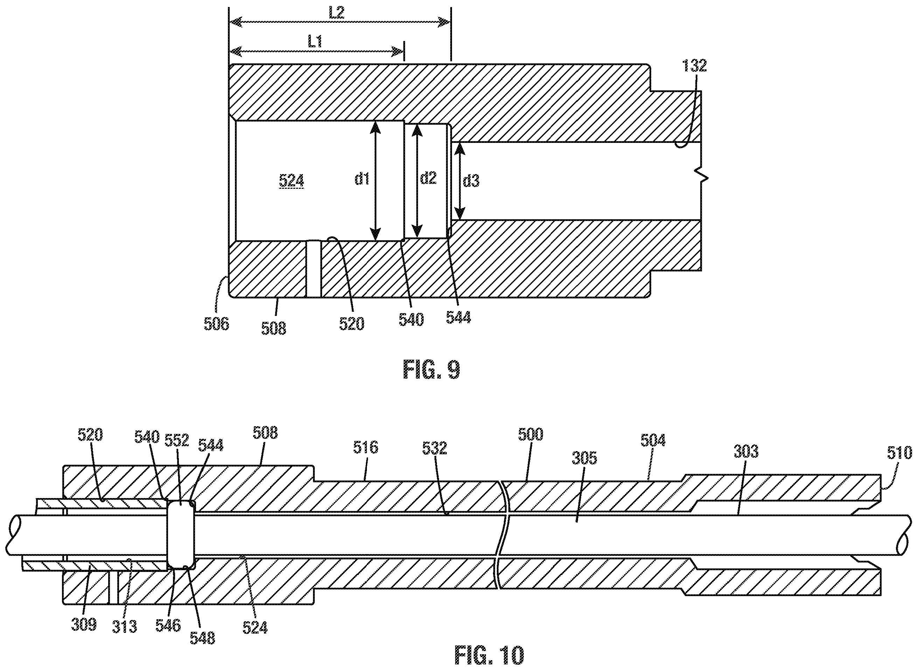

Referring to FIG. 9, a locating shoulder 540 is formed on the internal surface 520 of the head portion 508. The locating shoulder 540 defines a first stepdown transition in the internal bore 524. For example, the locating shoulder 540 steps down the diameter of the internal bore 524 from diameter d1 to diameter d2, where the diameter d1 is greater than the diameter d2. The locating shoulder 540 is offset from the distal end 506 of the carriage body 504 by a distance L1. The locating shoulder 540 has an annular face that is oriented towards the distal end 506 and may be referred to as "a distally facing annular shoulder" in some cases.

A gland shoulder 544 is formed on the internal surface 520 of the head portion 508. The gland shoulder 544 defines a second stepdown transition in the internal bore 524. For example, the gland shoulder 544 steps down the diameter of the internal bore 524 from diameter d2 to diameter d3, where the diameter d2 is greater than the diameter d3. The gland shoulder 544 is offset from the distal end 506 of the carriage body 504 by a distance L2 that is greater than the distance L1, which means that the gland shoulder 544 is located proximally to the locating shoulder 540. The gland shoulder 544 has an annular face that is oriented towards the distal end 506 and may be referred to as "a distally facing annular shoulder" in some cases.

FIG. 10 shows the shaft assembly 303 extending through the passage formed by the internal bore 524 and the central opening 532 such that the proximal end (or proximal face) of the outer shaft 309 is positioned within the internal bore 524. The proximal end of the outer shaft 309 forms a shoulder 546 that is in opposing relation to and distal relative to the gland shoulder 544. The outer shaft 309 can be secured to the head portion 508 of the carriage member 500 in this position (e.g., via fasteners, adhesive, and/or other means for coupling). An annular groove 548 (or gland) is defined within the internal bore 524 by the opposed shoulders 544, 546 and the portion of the internal surface 520 between the opposed shoulders 544, 546. The annular groove 548 can receive a seal member 552.

In some examples, the locating shoulder 540 can act as a stop surface for the proximal end of the outer shaft 309. In this case, the diameter d2 (shown in FIG. 9), which corresponds to the inner diameter of the locating shoulder 540, can be selected to be larger than an inner diameter of the outer shaft 309 at the proximal end of the outer shaft 309 such that when the proximal end of the outer shaft 309 abuts the locating shoulder 540, a portion of the proximal end of the outer shaft 309 forms the shoulder 546 at the first stepdown transition. As shown for example in FIG. 10, the shoulder 546 formed by the proximal end of the outer shaft 309 can be radially inward of the locating shoulder 540 at the first stepdown transition.

In other examples, the carriage body 504 can be formed without the locating shoulder 540, and the outer shaft 309 can be inserted into the internal bore 524 to a point at which the proximal face of the outer shaft 309 abuts the distal face of the seal member 522, which would at the same time form the distal end of the annular groove 548.

As illustrated by FIG. 10, the inner shaft 305 extending through the lumen of the outer shaft 309 passes through the portion of the internal bore 524 between the opposed gland shoulders 544, 546, which means that the annular groove 548 is disposed around a circumference of the inner shaft 305. Thus, the seal member 552 disposed in the annular groove 548 can form a seal between the inner shaft 305 and the internal surface 520 and at the proximal end of the outer shaft 309. The seal member 552 can cycle between dynamic sealing and static sealing. Dynamic sealing occurs when the seal member 552 slides along the inner shaft 305 as the carriage member 500 moves relative to the handle body 304 (shown in FIGS. 7B and 7C). In this manner, the seal member 552 can also be referred to as "a wiper seal." The seal member 552 can be any suitable seal (e.g., an O-ring).

The gland shoulder 544 forms the proximal end of the annular groove 548 (or the proximal gland shoulder), and the proximal end (or proximal face) of the outer shaft 309 forms the distal end of the annular groove 548 (or the distal gland shoulder). In some cases, the locating shoulder 540 can form a stop for the outer shaft 309. Forming the shoulders of the carriage body as stepped shoulders can, among other things, allow the carriage body 504 (or carriage member 500) to be molded as a single piece. The molding process can include forming a mold cavity for the carriage body and a core pin to form the internal bore including the locating and gland shoulders 540, 544. The core pin is secured within the mold cavity, and molten thermoplastic material is injected into the mold cavity to form the molded body. The stepped shoulders can, for example, allow the core pin to be easily removed from the distal end of the molded part. As such, the disclosed configuration simplifies both manufacture and assembly of the handle as one exemplary advantage.

Returning to FIG. 7C, the carriage member 500 is axially movable within the cavity 316 and relative to the handle body 304 by rotation of the drive member 320. In the example illustrated by FIG. 11A, the drive member 320 has a barrel portion 320a extending into the cavity 316 from the distal end 312 of the handle body 304 and a knob portion 320b projecting from the distal end 312 of the handle body 304. The barrel portion 320a has a ring member 332 that extends into a recess 336 in the handle body 304. A distal face of the ring member 332 can abut a proximal face of the recess 336 to limit movement of the drive member 320 in the distal direction.

The drive member 320 includes an internal surface 328 that defines an internal bore 340. The internal surface 328 includes an internal thread 344, which is complementary to the external threads 518 (shown in FIGS. 8A and 8B) on the head portion of the carriage member 500. As shown, the carriage member 500 extends into the internal bore 340 such that the external threads 518 on the head portion of the carriage member 500 are engaged with the internal thread 344 in the drive member 320.

Rotation of the knob portion 320b causes rotation of the drive member 320 relative to the handle body 304, which causes the carriage member 500 to move along the internal bore 340 of the drive member 320. The threads 344, 518 translate the rotary motion of the drive member 320 to a linear motion of the carriage member 500. However, other mechanisms besides a lead screw mechanism can be used to translate the carriage member 500 axially relative to the handle body 304.

Referring to FIGS. 11A and 11B, the handle body 304 can include flattened projections 348a, 348b (or guide members) extending into the cavity 316. The flattened projections 348a are received in the longitudinal slot 536a of the carriage member 500. The flattened projection 348b is received in the longitudinal slot 536b. The longitudinal slots 536a, 536b move along respective flattened projections 348a, 348b as the carriage member 500 moves axially within the cavity 316 and relative to the handle body 304. The flattened projections 348a, 348b are longitudinally aligned with the handle body 304 and cooperate with the longitudinal slots 536a, 536b to prevent rotation of the carriage member 500 when the drive member 320 is rotated.

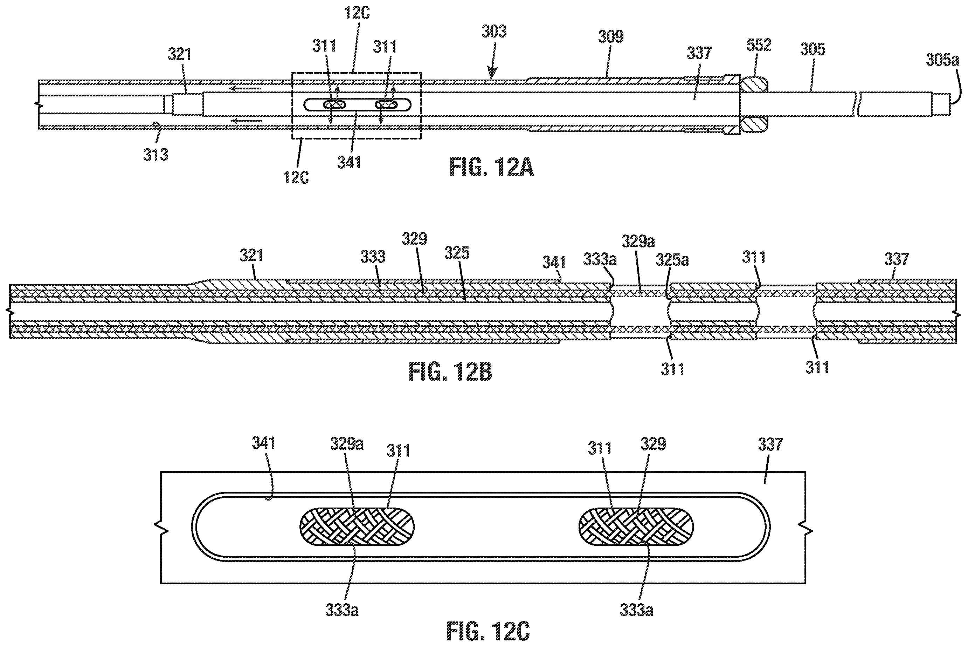

FIG. 12A shows a proximal portion of the shaft assembly 303 (i.e., the portion of the shaft assembly 303 immediately coupled to the handle). The proximal portion of the shaft assembly 303 includes a proximal portion of the outer shaft 309 and a proximal portion of the inner shaft 305 extending through the lumen 313 of the outer shaft 309. As previously described with respect to FIG. 11A, the proximal end of the outer shaft 309 is received within the carriage member 500, and the inner shaft 305 extends through the outer shaft 309 and through the carriage member. As shown in FIG. 12A, the proximal end portion of the inner shaft 305 includes the proximal end 305a that can be fluidly connected to the injection port 324 (shown in FIGS. 7A-7C and 11A) and the fluid ports 311 that allow fluid injected into the inner shaft 305 at the injection port to exit the inner shaft 305 and enter the lumen 313 of the outer shaft 309.

In one implementation, the inner shaft 305 includes a reinforced tube 321. In the example illustrated by FIG. 12B, the reinforced tube 321 can include an inner layer 325, a reinforcement layer 329 disposed over the inner layer 325, and an outer layer 333 disposed over the reinforcement layer 329. The inner layer 325, the reinforcement layer 329, and the outer layer 333 can be in the form of tubes extending substantially along the length of the inner shaft 305.

The reinforced tube 321 can be configured as a flexible tube to facilitate movement of the tube through the vasculature of a patient. The reinforcement layer 329 can be, for example, a braided tube, which can be made from metal wire (such as stainless-steel wire or Nitinol wire) or from synthetic fibers. The inner layer 325 and the outer layer 333 can be tubes made of a polymer material. Examples of suitable polymer materials include, but are not limited to, PEBAX^{®} elastomers, nylons, and polyurethane. The inner layer 325 and outer layer 333 can be made of the same material or of different materials. In some cases, the reinforced tube 321 can be made by extrusion.

The inner shaft 305 can include one or more fluid ports. The fluid ports are formed in the wall of the reinforced tube and can allow a flushing fluid to flow from the inner lumen of the inner shaft and into the lumen of the outer shaft 309. In this manner, the fluid ports 311 enable flushing of the inner shaft 305 and the outer shaft 309 from a single injection port rather than requiring the shafts to be separately flushed. Referring to FIGS. 12B and 12C, each fluid port 311 includes a first opening 325a in the inner layer 325, a second opening 333a in the outer layer 333 that is radially aligned with the first opening, and the pores (or openings) in the portion 329a of the reinforcement layer 329 between the two openings 325a, 333a. The openings 325a, 333a can have any suitable shape (e.g., oval as shown in FIGS. 12A and 12C, circular, square, or rectangular shape).

Any number of fluid ports 311 can be formed in the reinforced tube 321. For example, the illustrated reinforced tube 321 comprises four ports 311 (shown in FIG. 12B). When there are multiple fluid ports 311, various arrangements of the fluid ports 311 on the reinforced tube 321 are possible. For example, FIGS. 12A-12C show two of the fluid ports 311 spaced apart axially and aligned circumferentially along the reinforced tube 321. As depicted in FIG. 12B, the reinforced tube 321 also comprises two additional fluid ports 311 that are axially aligned and circumferentially spaced apart (e.g., by 180 degrees) from the fluid ports depicted in FIG. 12C. In another example, the fluid ports 311 can be spaced apart and/or staggered around the reinforced tube 321. For example, the fluid ports 311 can be spaced apart and staggered around the reinforced tube 321 to form a spiral pattern. In another example, the fluid ports can form an alternating-type pattern such that a first side of the tube comprises a plurality of ports (e.g., a first proximal port and a first distal port), and a second side of the tube (e.g., located 180 degrees from the first side) comprises a plurality of ports (e.g., a second proximal port and a second distal port), and the ports are arranged axially in the following manner moving proximal to distal: first proximal port, second proximal port, first distal port, second distal port.

The inner shaft 305 can, in some instances, include a cover tube 337 extending over a proximal portion of the reinforced tube 321. The cover tube 337 includes one or more windows 341 positioned to expose the fluid ports 311. The cover tube 337 is the part of the inner shaft 305 that contacts the seal member 552 (shown in FIG. 11A) when the inner shaft 305 extends through the carriage member 500 (shown in FIG. 11A). The cover tube 337 is preferably a rigid member that can support sliding of the seal member. The cover tube 337 preferably has a surface finish to provide a proper sealing surface to the seal member 552. The cover tube 337 can be made of metal or plastic. For example, the cover tube 337 can be made from stainless steel. The cover tube 337 can be secured to the reinforced tube 321 by any suitable method, such as by crimping, adhesive, etc.

The inner shaft 305 can be formed using any suitable method. One preferred method for forming the inner shaft 305 includes initially providing the reinforced tube 321 without the openings that form the fluid ports. The cover tube 337 with the one or more windows 341 formed therein is disposed over the reinforced tube 321 and secured to the outer layer 333 of the reinforced tube 321. The fluid ports 311 are then formed in the area of the reinforced tube 321 exposed through the window(s) 341 of the cover tube 337.

In one example, the openings that form each fluid port 311 are formed in the outer layer 333 and the inner layer 325 of the reinforced tube 321 by laser ablation. Advantageously, the laser beam used in the laser ablation can be configured to remove material from only portions of the outer layer 333 and the inner layer 325 of the reinforced tube 321, leaving the reinforcement layer 329 intact to maintain the tensile strength of the reinforced tube 321. In addition, the laser ablation process removes material by vaporizing the material, thereby reducing or eliminating potential particulate contamination of the delivery apparatus. Any film that settles on the surfaces of the tube can be flushed out.

Referring to FIGS. 11A and 12A, fluid (e.g., saline) can be injected into the inner shaft 305 through the injection port 324 for the purpose of flushing the inner shaft. The fluid will move through the lumen of the inner shaft 305. A portion of the fluid moving through the lumen of the inner shaft 305 will exit through the fluid ports 311 and enter the lumen 313 of the outer shaft 309, allowing flushing of the outer shaft. Thus, both the inner shaft 305 and the outer shaft 309 can be flushed using a single injection port. The seal member 552 forms a seal at the proximal end of the outer shaft 309 and prevents leakage of the fluid from the proximal end of the outer shaft. Later, during use of the delivery apparatus, the seal member 552 will also prevent leakage of blood from the proximal end of the outer shaft, thereby maintaining hemostasis.

Returning to FIGS. 6A-6F, the docking station 136 can be configured as a self-expanding docking station where the docking station 136 and the connector tabs 132 are naturally biased toward an expanded configuration. While the docking station 136 is attached to the delivery system, the docking station 136 is compressed to a smaller configuration (shown in FIG. 6B) for insertion and tracking through the vasculature. The compressed configuration of the docking station is held in place axially by the frame connector 400 (which is fixed relative to the inner shaft 305) and held in place radially by the outer shaft 309. The docking station 136 is therefore prevented from premature deployment by the frame connector 400 and the outer shaft 309. Once the docking station 136 is at the implantation location within the anatomy, the outer shaft 309 can be retracted to expose and deploy the docking station 136.

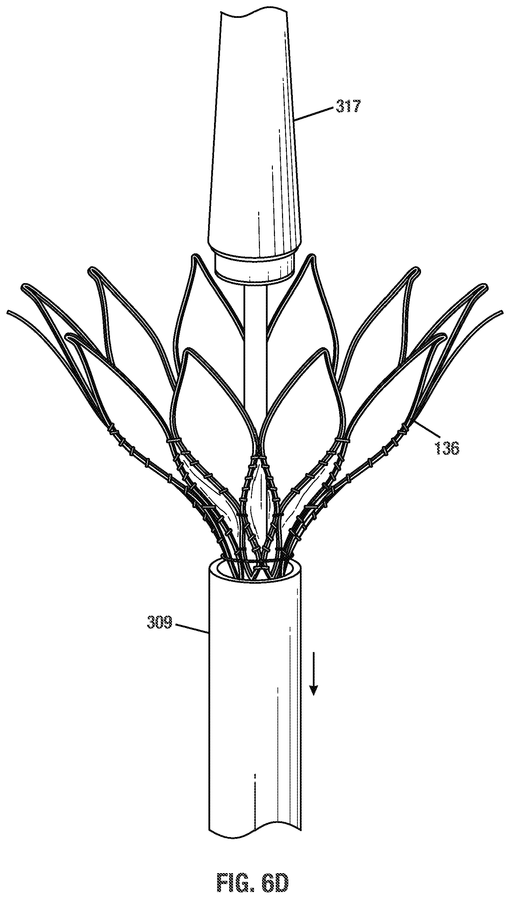

As the outer shaft 309 is retracted to expose the docking station 136, the distal portion of the docking station 136 expands (as shown, for example, in FIGS. 6C and 6D). In some cases, prior to completing retraction of the outer shaft 309, it may be desirable to reposition and/or retrieve the docking station 136. In this case, the outer shaft 309 may be extended again to recapture and recompress the docking station 136 in order to allow the docking station 136 to be repositioned and/or retrieved. However, the bias toward an expanded configuration can create an axial tension between the docking station and the frame connector. The axial tension can concentrate at the flanges of the connector tabs of the docking station as the outer shaft is extended distally over the docking station for recapture. Due to the relatively high forces during recapture and/or retrieval, the connector tabs of the docking station tend to move radially outwardly attempting to disengage from the frame connector 400. This can increase the force required to recapture the docking station. In extreme instances, the connector tabs can disengage from the connector, which can inhibit recompression and/or retrieval of the docking station.

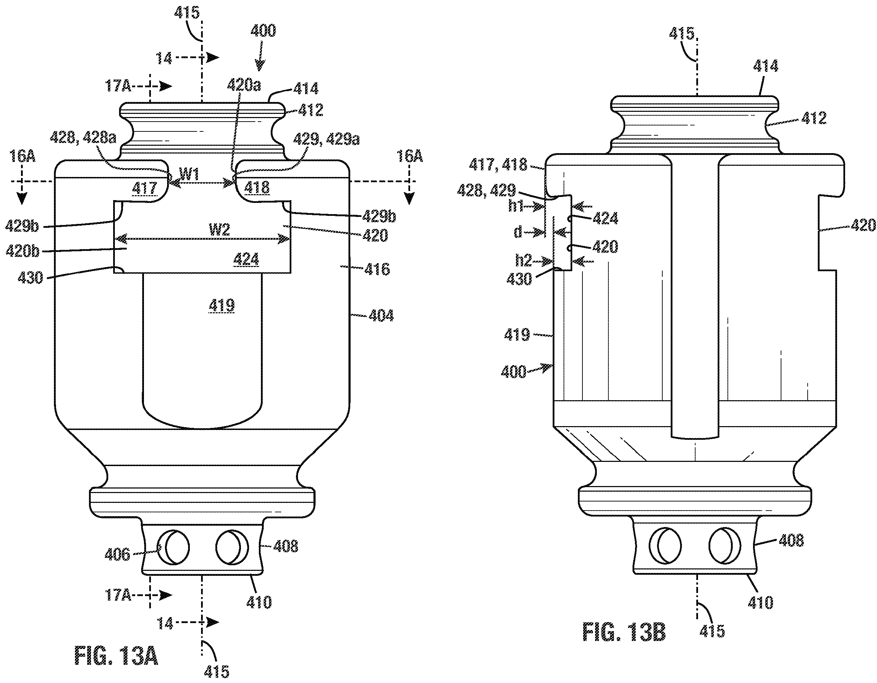

FIGS. 13A-17B illustrate an exemplary implementation of the frame connector 400 that can help retain the connector tabs in the radially-compressed configuration during recompression/retrieval of the docking station. Referring to FIGS. 13A and 13B, the frame connector 400 includes a connector body 404, a flange 408 attached to one end of the connector body 404, and a flange 412 attached to another end of the connector body 404. The flange 408 provides a proximal end 410 of the connector, and the flange 412 provides a distal end 414 of the connector. The frame connector 400 has a longitudinal axis 415 (or central axis) extending from the proximal end 410 to the distal end 414. The longitudinal axis 415 defines the axial direction of the connector.

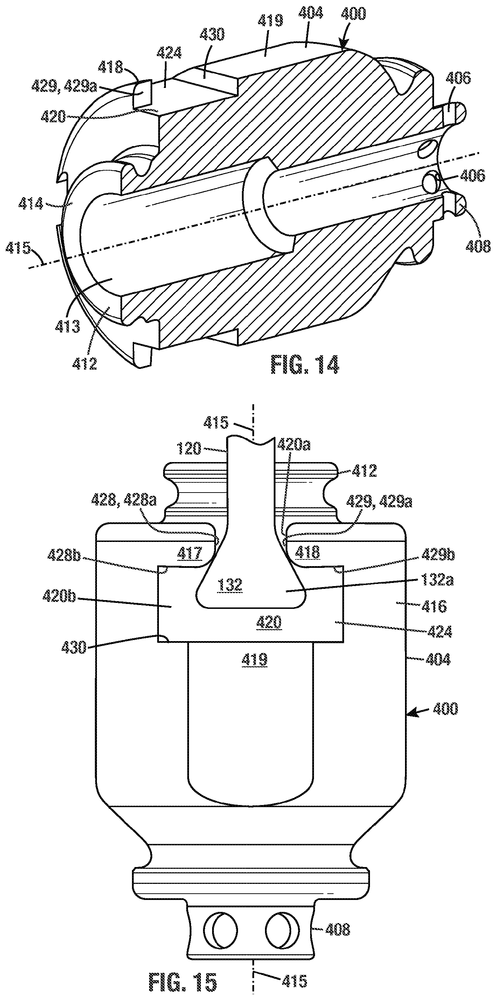

As shown in FIG. 14, the frame connector 400 has an internal bore 413 extending through the flanges 408, 412 and connector body 404 and along the longitudinal axis (415 in FIG. 13B). The internal bore 413 can receive a proximal portion of the inner shaft of the shaft assembly of the delivery apparatus. The flange 408 can include radial holes 406 that connect to the internal bore 413. As will be described later, the radial holes 406 can play a role when the frame connector 400 is fixed to the inner shaft of the shaft assembly (e.g., by an over-molding process).

Returning to FIGS. 13A and 13B, the connector body 404 includes an exterior with an exterior surface 416 and one or more recesses 420. Each of the recesses 420 can receive one of the connector tabs of the docking station. In the implementation illustrated by FIGS. 13A-17B, two recesses 420 are formed in diametrically opposed positions on the exterior of the connector body 404. In general, when a plurality of recesses 420 are formed on the exterior of the connector body 404, the recesses 420 can be formed in angularly (which may also be referred to as "circumferentially") spaced apart locations along the exterior of the connector body 404 (i.e., distributed along a circumference of the connector body 404).

Referring still to FIGS. 13A and 13B, each recess 420 can be a recessed slot having a first slot portion 420a and a second slot portion 420b arranged to form a "T" shape. As shown, the first slot portion 420a is generally aligned with the longitudinal axis 415 of the connector and is generally perpendicular to the second slot portion 420b. The first slot portion 420a has a first width W1, and the second slot portion 420b has a second width W2. The second width W2 is greater than the first width W1, which means that the recess 420 transitions from a larger width slot portion 420b to a smaller width slot portion 420a. As shown in FIG. 15, the recess 420 is open at the exterior surface 416 so that a connector tab 132 having a flared portion 132a can be positioned in the recess from the exterior surface 416.

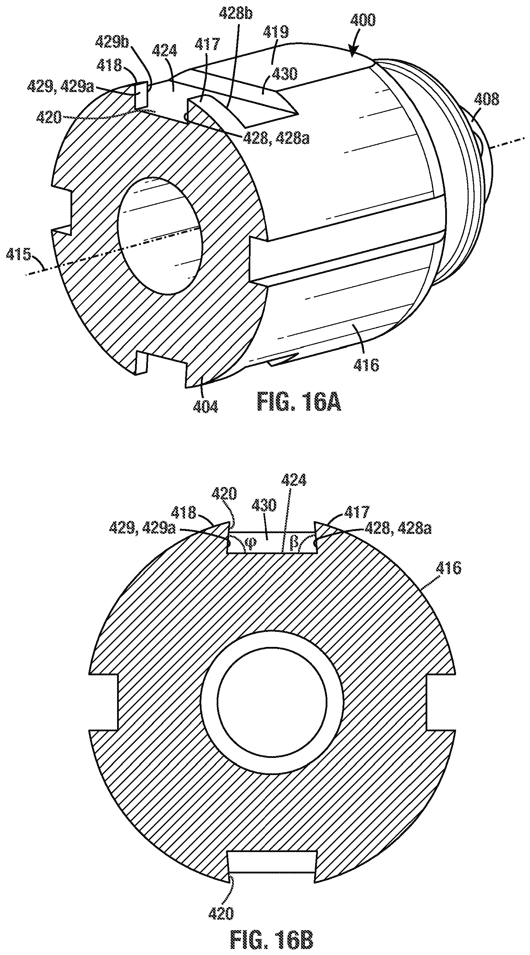

Referring to FIGS. 13A and 16A, each recess 420 has a recess floor 424, opposite side walls 428, 429, and an end wall 430. The side walls 428, 429 project from opposite sides of the recess floor 424. The side wall 428 is connected to a portion 417 of the exterior surface 416. The side wall 429 is connected to a portion 418 of the exterior surface 416. The end wall 430 projects from an end of the recess floor 424 and is connected to a portion 419 of the exterior surface 416. The recess floor 424 is on a different plane compared to the surface portions 417, 418, 419. In particular, the recess floor 424 is recessed (or radially inward) relative to the surface portions 417, 418, 419, as shown more clearly in FIG. 16A.

In one example, the surface portions 417, 418 are on the same plane but on a different plane compared to the surface portion 419. For example, as shown in FIG. 13B, each of the surface portions 417, 418 can be radially outward of the surface portion 419 by an offset distance d. Stated differently, the height h1 of the side walls 428, 429 relative to the recess floor 424 can be greater than the height h2 of the end wall 430 relative to the recess floor 424. Since the connector tab that is received in the recess 420 will contact the side walls 428, 429, the height of the side walls 428, 429 can be selected to provide sufficient engagement surfaces for the connector tab.

A first portion 428a of the side wall 428 and a first portion 429a of the side wall 429 form opposite sides of the first slot portion 420a (in FIG. 13A) of the recess 420. The end wall 430 is longitudinally displaced from the first and second walls 428, 429 by a distance that determines the height of the second slot portion 420b (in FIG. 13A) of the recess 420. A second portion 428b of the side wall 428 and a second portion 429b of the side wall 429 are in opposing relation to the end wall 430. The end wall 430 and the second portions 428b, 429b of the side walls 428, 429 form opposite ends of the second slot portion 420b of the recess 420.

FIG. 15 shows a connector tab 132 of a docking station positioned within a recess 420 of the frame connector 400 prior to deployment of the docking station at an implantation location. The connector tab 132 can be formed at an apex of a strut 120 of the frame of the docking station as previously described. In the example illustrated by FIG. 15, the connector tab 132 has a flared portion 132a that sits in the second slot portion 420b and engages the side walls 428, 429. The flared portion 132a engages the side walls 428, 429 because the flared portion 132a is wider than the first slot portion 420a. When the flared portion 132a engages the side walls 428, 429 as shown, the connector tab 132 is prevented from being pulled axially through the first slot portion 420a.

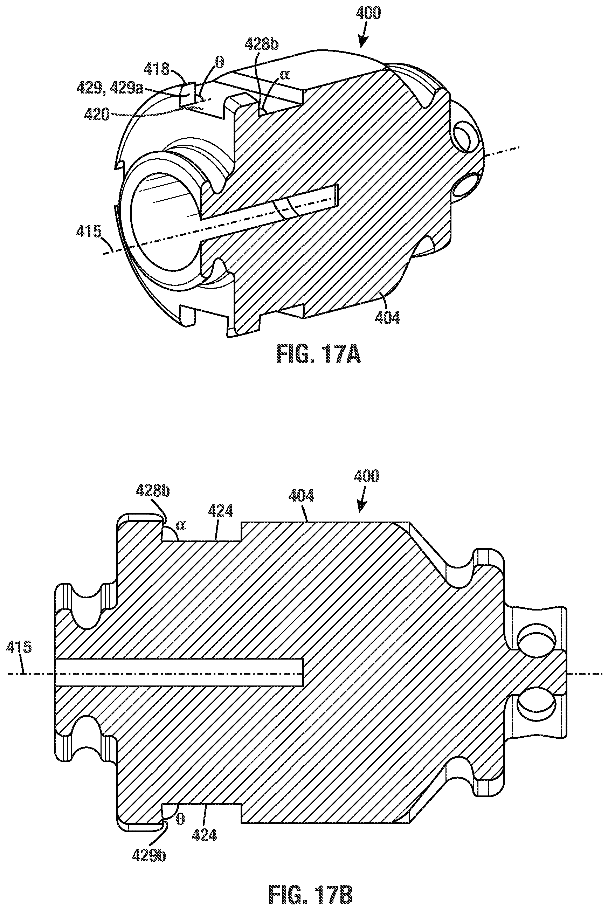

To help retain the connector tab 132 in the radially-compressed configuration and thus its connection with the frame connector 400 when axial tension is created between the docking station and the frame connector, the second portions 428b, 429b of the side walls 428, 429 are formed as undercut walls, which means that there is a space or recess underneath each of the second portions 428b, 429b (or a space or recess between each of the second portions 428b, 429b and the recess floor 424). As illustrated in FIGS. 17A and 17B, the second portions 428b, 429b, which are formed as undercut walls, are inclined relative to the recess floor 424 (i.e., the second portions 428b, 429b are not perpendicular to the recess floor 424). The angle α between the second portion 428b and the recess floor 424 is less than 90 degrees, and the angle θ between the second portion 429b and the recess floor 424 is less than 90 degrees. In some examples, each of the angles α and θ can be in a range of 45-89.9 degrees. In other examples, each of the angles α and θ can be in a range of 75-89.9 degrees. In one preferred example, each of the angles α and θ can be in a range of 81-86 degrees. The angles α and θ can be the same or can be different.

When the frame connector 400 as illustrated by FIGS. 17A and 17B is used to axially restrain the docking station 136, the tensile force created by the bias of the docking station to the expanded configuration pulls the flared portion (132a in FIG. 15) of the connector tab axially against the second portions 428b, 429b. The undercut in the second portions 428b, 429b translates a portion of the tensile force into a radial force that pushes the connector tab radially inwardly toward the central axis of the frame connector 400, thereby improving retention characteristics of the docking station prior to deployment of the docking station. It has been found that each of the angles α, θ between the second portions 428b, 429b and the recess floor 424 in a range of 81-86 degrees (in certain instances) improves securement of the docking station to the delivery system when the outer shaft is extended during recapture of the docking station.

Returning to FIGS. 13A and 16A, the first portions 428a, 429a can be formed as undercut walls, which means that there is a space or recess underneath each of the first portions 428a, 429a (or a space or recess between each of the first portions 428a, 429a and the recess floor 424). As illustrated in FIG. 16B, the first portions 428a, 428b as undercut walls are inclined relative to the recess floor 424 (i.e., the first portions 428a, 429b are not perpendicular to the recess floor 424). The angle β between the first portion 428a and the recess floor 424 is less than 90 degrees, and the angle φ between the first portion 429a and the recess floor 424 is less than 90 degrees. In some examples, each of the angles β and φ can be in a range of 45-89.9 degrees. In other examples, each of the angles β and φ can be in a range of 75-89.9 degrees. In one example, each of the angles β and φ can be in a range of 81-86 degrees. The angles β and φ can be the same or can be different. In some examples, the angles β and/or φ can be the same as the angles α and/or θ. In other examples, the angles β and/or φ can be different than the angles α and/or θ.

Returning to FIG. 13A, each of the side walls 428, 429 includes a corner where the first slot portion 420a is connected to the second slot portion 420b. These corners can be rounded and can have undercuts such that an undercut extends underneath the entire length of each of the side walls 428, 429. The edges where the side walls 428, 429 meet the exterior surface portions 417, 418 can be similarly rounded.

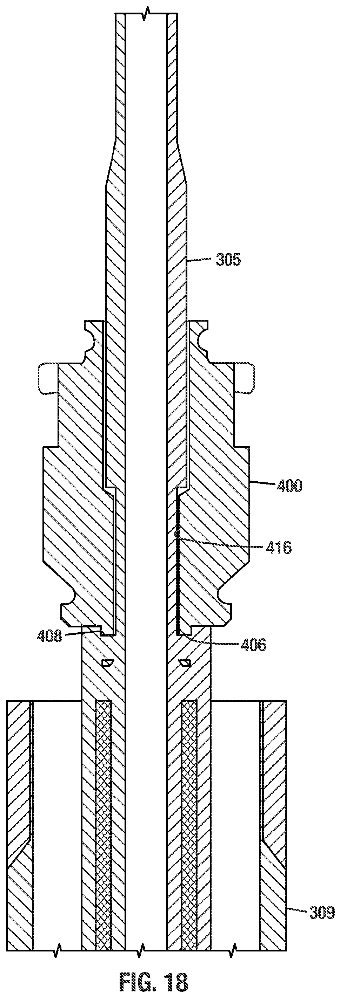

Referring to FIG. 18, one preferred method of coupling the frame connector 400 to a distal portion of the inner shaft 305 (shown in FIG. 5A) is by an over-molding process. During the over-molding process, the radial holes 406 in the flange 408 can receive flow of injected material. The material in the radial holes 406, when solidified, can anchor the frame connector 400 to the inner shaft 305. FIG. 18 shows the inner shaft 305 extending through the lumen of the outer shaft 309. The frame connector 400 is sized relative to the outer shaft 309 such that the outer shaft 309 can be extended over the frame connector 400 and over a docking station disposed around a portion of the inner shaft 305 distal to the frame connector 400.

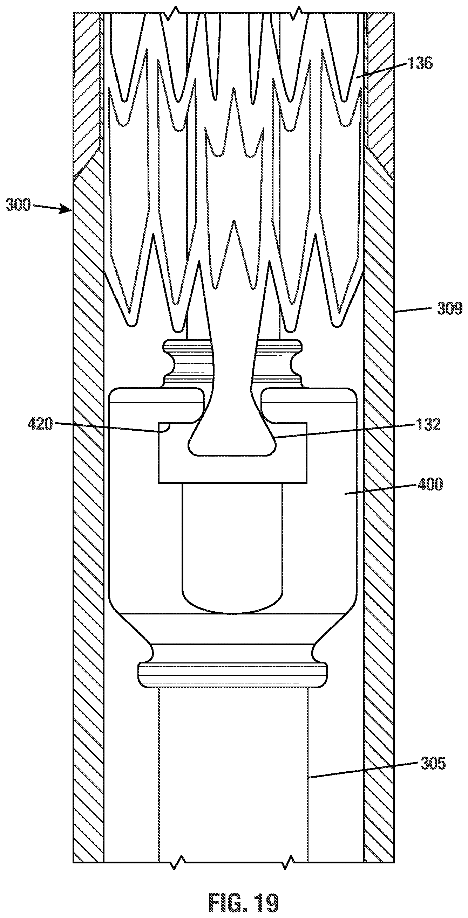

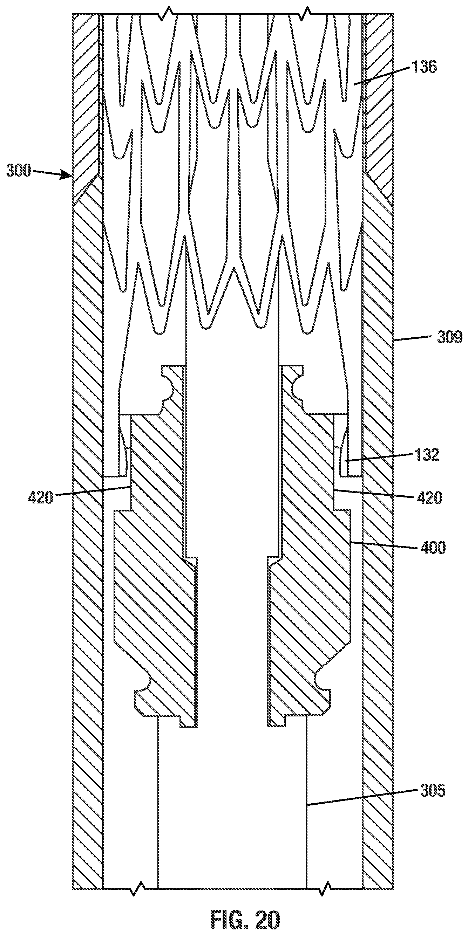

FIGS. 19 and 20 illustrate a portion of the delivery apparatus 300 including the docking station 136 in a compressed configuration. The outer shaft 309 is extended to encapsulate the docking station 136. Each of the connector tabs 132 of the docking station 136 is disposed in a respective recess 420 of the frame connector 400 and engaged with the side walls of the recess 420. The docking station 136 is held in place axially by the frame connector 400 and radially by the outer shaft 309. It should be understood that only a portion of the delivery apparatus is shown in FIGS. 19 and 20. The remaining portions of the delivery apparatus (e.g., the portion that extends to the nosecone, the portion that is coupled to the handle, the nosecone, and the handle) are visible in FIGS. 5A.

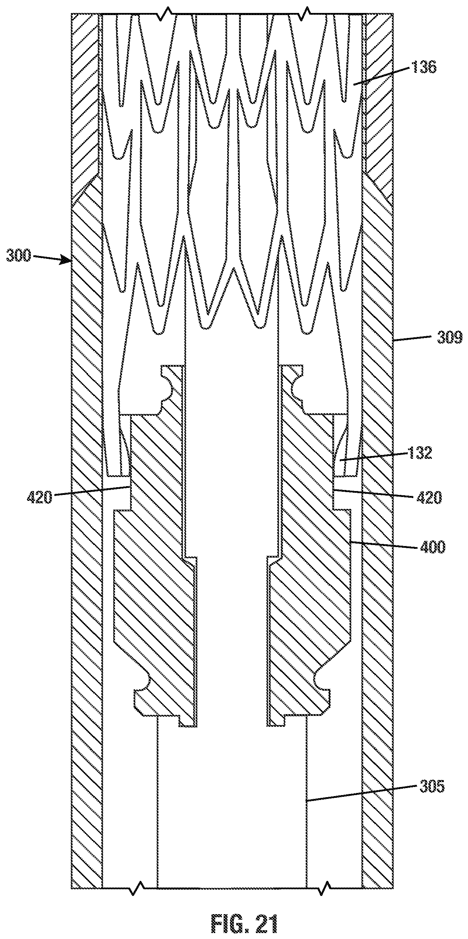

A delivery assembly that is configured as shown in FIGS. 19 and 20 can be inserted into a patient's body and advanced through the patient's vasculature to an implantation location. At the implantation location, the outer shaft 309 can be retracted to expose the docking station 136 and deploy the docking station (as illustrated in FIGS. 6C-6F). During recapture of the docking station 136, the inner shaft 305 can be under high tensile loads while the outer shaft 309 is extended to cover docking station 136. The undercut in the side walls of the recess 420 can translate the tensile force acting on the respective connector tab 132 to a radial force that pushes the connector tab 132 inwardly toward the central axis of the frame connector 400, as illustrated in FIG. 21, thereby retaining the connection between the delivery apparatus and the docking station.

## Claims

1. A delivery apparatus (300) comprising:
a handle body (304) having a proximal end (308), a distal end (312), a longitudinal axis (315) extending between the proximal end (308) and the distal end (312), and a cavity (316) disposed between the proximal end (308) and the distal end (312);
a carriage member (500) disposed within the cavity (316) and axially movable relative to the handle body (304) in a direction parallel to the longitudinal axis of the handle body (304), the carriage member (500) having an internal surface (520) defining an internal bore (524) and a gland shoulder (544) integrally formed with the internal surface (520) and defining a stepdown transition in the internal bore (524);
an outer shaft (309) having a proximal end positioned within the internal bore (524) of the carriage member (500) and in opposing relation to the gland shoulder (544), wherein the proximal end of the outer shaft (309), the gland shoulder (544) of the carriage member (500), and a portion of the internal surface (520) of the carriage member (500) adjacent to the stepdown transition define an annular groove (548);
an inner shaft (305) extending through a lumen (313) of the outer shaft (309) and fixed relative to the handle body (304); and
a seal member (552) disposed around the inner shaft (305) and within the annular groove (548), wherein the seal member (552) is positioned to form a seal between the carriage member (500) and the inner shaft (305) and at the proximal end of the outer shaft (309).

2. The delivery apparatus of claim 1, wherein the carriage member (500) further comprises a locating shoulder (540) integrally formed with the internal surface (520) and axially displaced from the gland shoulder (544), and wherein the proximal end of the outer shaft (309) abuts the locating shoulder (540) of the carriage member (500) to define the annular groove (548).

3. The delivery apparatus of claim 1 or 2, wherein the gland shoulder (544) is an annular shoulder.

4. The delivery apparatus of any one of claims 1 to 3, wherein the carriage member (500) is a one-piece molded body.

5. The delivery apparatus of any one of claims 1 to 4, wherein the outer shaft (309) is coupled to the carriage member (500) and movable relative to the handle body (304) by the carriage member (500).

6. The delivery apparatus of any one of claims 1 to 5, further comprising:
a first guide member formed in the carriage member (500); and
a second guide member formed in the handle body (304),
wherein the first and second guide members guide axial movement of the carriage member (500) along the longitudinal axis and restrict relative rotational movement between the carriage member (500) and the handle body (304).

7. The delivery apparatus of any one of claims 1 to 5, further comprising:
opposed first and second longitudinal slots (536a, 536b) formed in the carriage member (500); and
opposed first and second flattened projections (348a, 348b) formed on the handle body (304),
wherein the opposed first and second flattened projections (348a, 348b) respectively extend into the opposed first and second longitudinal slots (536a, 536b) to guide axial movement of the carriage member (500) along the longitudinal axis and inhibit relative rotational movement between the carriage member (500) and the handle body (304).

8. The delivery apparatus of any one of claims 1 to 7, wherein the carriage member (500) comprises a head portion (508) and a stem portion (512), and wherein the internal bore (524) is formed in the head portion (508).

9. The delivery apparatus of claim 8, wherein the stem portion (512) comprises a central opening (532) that is connected to the internal bore (524), and wherein the internal bore (524) and the central opening (532) form a passage extending along a length of the carriage member (500).

10. The delivery apparatus of claim 9, wherein the head portion (508) comprises an external threaded surface (518).

11. The delivery apparatus of claim 10, further comprising:
a drive member (320) rotatably supported within the handle body (304), the drive member (320) having an internal threaded surface (344) that is threadedly engaged with the external threaded surface (518) of the carriage member (500),
wherein rotation of the drive member (320) relative to the handle body (304) results in the carriage member (500) moving relative to the handle body (304) along the longitudinal axis.

12. The delivery apparatus of claim 11, wherein the drive member (320) comprises a barrel portion received partially within the cavity (316) of the handle body (304) and a knob portion (320b) operable to rotate the barrel portion (320a) relative to the handle body (304), and wherein the internal threaded surface is formed in the barrel portion and the knob portion (320b).

13. The delivery apparatus of any one of claims 1 to 12, further comprising an injection port (324) disposed at the proximal end (308) of the handle body (304), the injection port (324) fluidly connected to a lumen of the inner shaft (305).

14. A delivery assembly comprising:
the delivery apparatus (300) of any one of claims 1 to 13; and
an expandable docking station (136) releasably coupled to the delivery apparatus (300), wherein the expandable docking station (136) is configured to receive a prosthetic heart valve.

## Patentansprüche

1. Zuführvorrichtung (300), umfassend:
einen Griffkörper (304) mit einem proximalen Ende (308), einem distalen Ende (312), einer Längsachse (315), die sich zwischen dem proximalen Ende (308) und dem distalen Ende (312) erstreckt, und einem Hohlraum (316), der zwischen dem proximalen Ende (308) und dem distalen Ende (312) angeordnet ist;
ein Schlittenelement (500), das innerhalb des Hohlraums (316) angeordnet und relativ zum Griffkörper (304) in einer Richtung parallel zur Längsachse des Griffkörpers (304) axial bewegbar ist, wobei das Schlittenelement (500) eine innere Oberfläche (520) aufweist, die eine Innenbohrung (524) definiert, und eine Dichtungsschulter (544), die integral mit der inneren Oberfläche (520) ausgebildet ist und einen Stufenübergang in der Innenbohrung (524) definiert;
einen Außenschaft (309) mit einem proximalen Ende, das innerhalb der Innenbohrung (524) des Schlittenelements (500) und in gegenüberliegender Beziehung zur Dichtungsschulter (544) positioniert ist, wobei das proximale Ende des Außenschafts (309), die Dichtungsschulter (544) des Schlittenelements (500) und ein Abschnitt der inneren Oberfläche (520) des Schlittenelements (500) benachbart zum Stufenübergang eine ringförmige Nut (548) definieren;
einen Innenschaft (305), der sich durch ein Lumen (313) des Außenschafts (309) erstreckt und relativ zum Griffkörper (304) fixiert ist; und
ein Dichtungselement (552), das um den Innenschaft (305) und innerhalb der ringförmigen Nut (548) angeordnet ist, wobei das Dichtungselement (552) so positioniert ist, dass es eine Abdichtung zwischen dem Schlittenelement (500) und dem Innenschaft (305) sowie am proximalen Ende des Außenschafts (309) bildet.

2. Zuführvorrichtung gemäß Anspruch 1, wobei das Schlittenelement (500) ferner eine Positionierschulter (540) umfasst, die integral mit der inneren Oberfläche (520) ausgebildet und axial von der Dichtungsschulter (544) versetzt ist, und wobei das proximale Ende des Außenschafts (309) an der Positionierschulter (540) des Schlittenelements (500) anliegt, um die ringförmige Nut (548) zu definieren.

3. Zuführvorrichtung gemäß Anspruch 1 oder 2, wobei die Dichtungsschulter (544) eine ringförmige Schulter ist.

4. Zuführvorrichtung gemäß einem der Ansprüche 1 bis 3, wobei das Schlittenelement (500) ein einteiliger Formkörper ist.

5. Zuführvorrichtung gemäß einem der Ansprüche 1 bis 4, wobei der Außenschaft (309) mit dem Schlittenelement (500) gekoppelt und durch das Schlittenelement (500) relativ zum Griffkörper (304) bewegbar ist.

6. Zuführvorrichtung gemäß einem der Ansprüche 1 bis 5, ferner umfassend:
ein erstes Führungselement, das in dem Schlittenelement (500) ausgebildet ist; und
ein zweites Führungselement, das in dem Griffkörper (304) ausgebildet ist,
wobei das erste und das zweite Führungselement die axiale Bewegung des Schlittenelements (500) entlang der Längsachse führen und die relative Drehbewegung zwischen dem Schlittenelement (500) und dem Griffkörper (304) einschränken.

7. Zuführvorrichtung gemäß einem der Ansprüche 1 bis 5, ferner umfassend:
einen ersten und einen zweiten gegenüberliegenden Längsschlitz (536a, 536b), die in dem Schlittenelement (500) ausgebildet sind; und
einen ersten und einen zweiten gegenüberliegenden abgeflachten Vorsprung (348a, 348b), die an dem Griffkörper (304) ausgebildet sind,
wobei sich der erste und der zweite gegenüberliegende abgeflachte Vorsprung (348a, 348b) jeweils in den ersten und den zweiten gegenüberliegenden Längsschlitz (536a, 536b) erstrecken, um die axiale Bewegung des Schlittenelements (500) entlang der Längsachse zu führen und relative Drehbewegung zwischen dem Schlittenelement (500) und dem Griffkörper (304) zu verhindern.

8. Zuführvorrichtung gemäß einem der Ansprüche 1 bis 7, wobei das Schlittenelement (500) einen Kopfabschnitt (508) und einen Schaftabschnitt (512) umfasst, und wobei die Innenbohrung (524) in dem Kopfabschnitt (508) ausgebildet ist.

9. Zuführvorrichtung gemäß Anspruch 8, wobei der Schaftabschnitt (512) eine zentrale Öffnung (532) umfasst, die mit der Innenbohrung (524) verbunden ist, und wobei die Innenbohrung (524) und die zentrale Öffnung (532) einen Durchgang bilden, der sich entlang einer Länge des Schlittenelements (500) erstreckt.

10. Zuführvorrichtung gemäß Anspruch 9, wobei der Kopfabschnitt (508) eine Außengewindefläche (518) umfasst.

11. Zuführvorrichtung gemäß Anspruch 10, ferner umfassend:
ein Antriebselement (320), das drehbar innerhalb des Griffkörpers (304) gelagert ist, wobei das Antriebselement (320) eine Innengewindefläche (344) aufweist, die mit der Außengewindefläche (518) des Schlittenelements (500) in Gewindeeingriff steht,
wobei eine Drehung des Antriebselements (320) relativ zum Griffkörper (304) dazu führt, dass sich das Schlittenelement (500) relativ zum Griffkörper (304) entlang der Längsachse bewegt.

12. Zuführvorrichtung gemäß Anspruch 11, wobei das Antriebselement (320) einen Zylinderabschnitt, der teilweise innerhalb des Hohlraums (316) des Griffkörpers (304) aufgenommen ist, und einen Knopfabschnitt (320b) umfasst, der betätigbar ist, um den Zylinderabschnitt (320a) relativ zum Griffkörper (304) zu drehen, und wobei die Innengewindefläche in dem Zylinderabschnitt und dem Knopfabschnitt (320b) ausgebildet ist.

13. Zuführvorrichtung gemäß einem der Ansprüche 1 bis 12, ferner umfassend einen Injektionsport (324), der an dem proximalen Ende (308) des Griffkörpers (304) angeordnet ist, wobei der Injektionsport (324) fluidisch mit einem Lumen des Innenschafts (305) verbunden ist.

14. Zuführanordnung, umfassend:
die Zuführvorrichtung (300) gemäß einem der Ansprüche 1 bis 13; und
eine expandierbare Docking-Station (136), die lösbar mit der Zuführvorrichtung (300) gekoppelt ist, wobei die expandierbare Docking-Station (136) dazu ausgebildet ist, eine Herzklappenprothese aufzunehmen.

## Revendications

1. Appareil de pose (300), comprenant :
un corps de poignée (304) présentant une extrémité proximale (308), une extrémité distale (312), un axe longitudinal (315) s'étendant entre l'extrémité proximale (308) et l'extrémité distale (312) et une cavité (316) disposée entre l'extrémité proximale (308) et l'extrémité distale (312) ;
un élément chariot (500) disposé à l'intérieur de la cavité (316) et mobile axialement par rapport au corps de poignée (304) dans une direction parallèle à l'axe longitudinal du corps de poignée (304), l'élément chariot (500) présentant une surface interne (520) définissant un trou interne (524) et un épaulement de presse-étoupe (544) formé d'un seul tenant avec la surface interne (520) et définissant une transition décroissante dans le trou interne (524) ;
une tige externe (309) présentant une extrémité proximale positionnée à l'intérieur du trou interne (524) de l'élément chariot (500) et en relation opposée par rapport à l'épaulement de presse-étoupe (544), l'extrémité proximale de la tige externe (309), l'épaulement de presse-étoupe (544) de l'élément chariot (500) et une partie de la surface interne (520) de l'élément chariot (500) adjacente à la transition décroissante définissant une rainure annulaire (548) ;
une tige interne (305) s'étendant à travers une lumière (313) de la tige externe (309) et fixée par rapport au corps de poignée (304) ; et
un élément d'étanchéité (552) disposé autour de la tige interne (305) et à l'intérieur de la rainure annulaire (548), l'élément d'étanchéité (552) étant positionné pour former un joint d'étanchéité entre l'élément chariot (500) et la tige interne (305) et au niveau de l'extrémité proximale de la tige externe (309).

2. Appareil de pose selon la revendication 1, l'élément chariot (500) comprenant en outre un épaulement de positionnement (540) formé d'un seul tenant avec la surface interne (520) et déplacé axialement à partir de l'épaulement de presse-étoupe (544) et l'extrémité proximale de la tige externe (309) venant en butée contre l'épaulement de positionnement (540) de l'élément chariot (500) afin de définir la rainure annulaire (548).

3. Appareil de pose selon la revendication 1 ou 2, l'épaulement de presse-étoupe (544) étant un épaulement annulaire.

4. Appareil de pose selon l'une quelconque des revendications 1 à 3, l'élément chariot (500) étant un corps moulé d'une seule pièce.

5. Appareil de pose selon l'une quelconque des revendications 1 à 4, la tige externe (309) étant accouplée à l'élément chariot (500) et mobile par rapport au corps de poignée (304) par l'élément chariot (500).

6. Appareil de pose selon l'une quelconque des revendications 1 à 5, comprenant en outre :
un premier élément de guidage formé dans l'élément chariot (500) ; et
un second élément de guidage formé dans le corps de poignée (304),
les premier et second éléments de guidage guidant le déplacement axial de l'élément chariot (500) le long de l'axe longitudinal et limitant le déplacement de rotation relatif entre l'élément chariot (500) et le corps de poignée (304).

7. Appareil de pose selon l'une quelconque des revendications 1 à 5, comprenant en outre :
des première et seconde fentes longitudinales opposées (536a, 536b) formées dans l'élément chariot (500) ; et
des première et seconde saillies aplaties opposées (348a, 348b) formées sur le corps de poignée (304),
les première et seconde saillies aplaties opposées (348a, 348b) s'étendant respectivement dans les première et seconde fentes longitudinales opposées (536a, 536b) afin de guider le déplacement axial de l'élément chariot (500) le long de l'axe longitudinal et d'empêcher un déplacement de rotation relatif entre l'élément chariot (500) et le corps de poignée (304).

8. Appareil de pose selon l'une quelconque des revendications 1 à 7, l'élément chariot (500) comprenant une partie tête (508) et une partie tige (512) et le trou interne (524) étant formé dans la partie tête (508).

9. Appareil de pose selon la revendication 8, la partie tige (512) comprenant une ouverture centrale (532) qui est reliée au trou interne (524) et le trou interne (524) et l'ouverture centrale (532) formant un passage s'étendant le long d'une longueur de l'élément chariot (500).

10. Appareil de pose selon la revendication 9, la partie tête (508) comprenant une surface filetée externe (518).

11. Appareil de pose selon la revendication 10, comprenant en outre :
un élément d'entraînement (320) supporté de manière rotative à l'intérieur du corps de poignée (304), l'élément d'entraînement (320) présentant une surface filetée interne (344) qui est en prise par filetage avec la surface filetée externe (518) de l'élément chariot (500),
la rotation de l'élément d'entraînement (320) par rapport au corps de poignée (304) entraînant le déplacement de l'élément chariot (500) par rapport au corps de poignée (304) le long de l'axe longitudinal.

12. Appareil de pose selon la revendication 11, l'élément d'entraînement (320) comprenant une partie cylindre logée partiellement à l'intérieur de la cavité (316) du corps de poignée (304) et une partie bouton (320b) utilisable pour faire tourner la partie cylindre (320a) par rapport au corps de poignée (304) et la surface filetée interne étant formée dans la partie cylindre et la partie bouton (320b).

13. Appareil de pose selon l'une quelconque des revendications 1 à 12, comprenant en outre un orifice d'injection (324) disposé au niveau de l'extrémité proximale (308) du corps de poignée (304), l'orifice d'injection (324) étant en communication fluidique avec une lumière de la tige interne (305).

14. Ensemble de pose comprenant :
l'appareil de pose (300) selon l'une quelconque des revendications 1 à 13 ;
et
une station d'ancrage (136) déployable accouplée de manière amovible à l'appareil de pose (300), la station d'ancrage déployable (136) étant conçue pour recevoir une valvule cardiaque prothétique.
